(19) Europäisches Patentamt
European Patent Office
Office européen des brevets

(11) EP 3 556 748 A1

(12) EUROPEAN PATENT APPLICATION

(43) Date of publication:
**23.10.2019 Bulletin 2019/43**

(51) Int Cl.:
***C07D 251/24*** *(2006.01)* ***A61K 31/53*** *(2006.01)*

(21) Application number: **18168380.6**

(22) Date of filing: **20.04.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **BASF SE**
**67056 Ludwigshafen am Rhein (DE)**

(72) Inventors:
- **Ehlis, Thomas**
  **4133 Schweizhalle (Muttenz) (CH)**
- **Burger, Thomas**
  **4133 Schweizhalle (Muttenz) (CH)**
- **Grumelard, Julie**
  **79639 Grenzach-Wyhlen (DE)**

(74) Representative: **BASF IP Association**
**BASF SE**
**G-FLP-C006**
**67056 Ludwigshafen (DE)**

(54) **HYDROXYPHENYL-TRIAZINE UV ABSORBERS**

(57) Disclosed is a UV absorber mixture comprising at least two compounds which refer to the reaction product of an oxirane mixture and a hydroxyphenyl-triazine compound.

The reaction product of according to the present invention represents UV radiation absorbing mixtures with excellent solubility and photostability properties.

EP 3 556 748 A1

## Description

Numerous sunscreen compositions are commercially available with varying ability to shield the human skin from ultraviolet light. However, numerous challenges still exist for sunscreen compositions providing strong UV radiation protection (high SPF) and minimized skin permeation. Hydroxyphenyl-triazines like Tinosorb S (BASF) can provide broad UV spectrum protection. However, due to their limited solubility in cosmetic solvents, the practical applicability is restricted.

It was now found, that the reaction product of sparingly soluble triazine UV absorber chromophores with mixtures of oxiranes represent UV radiation absorbing mixtures with excellent solubility and photostability properties. The reaction products are based on the same intermediates, which are already used for other commercially available UV filters.

Therefore, the present invention refers to a UV absorber mixture comprising at least two compounds which refer to the reaction product of an oxirane mixture and a hydroxyphenyl-triazine compound.

The oxiranes used in the present invention, preferably correspond to formula

(1a) 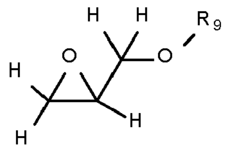 ,

wherein

Rg is $C_1$-$C_{18}$alkyl; or $C_3$-$C_{12}$cycloalkyl.

Preferably, the oxirane mixture comprises at least 2 different oxiranes of formula (1a), and more preferably at least 3 different oxiranes of formula (1a).

Preferably, the reaction product according to the present invention represents a mixture of UV absorbers which corresponds to formula

(1) 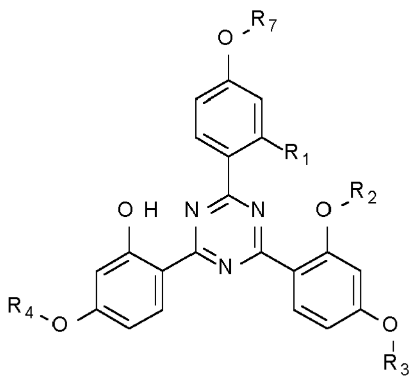 ,

wherein

$R_1$ is hydrogen; or -O-$R_6$;

$R_2$, $R_3$, $R_4$ and $R_6$, independently from each other are hydrogen; or a radical of formula

(1b) 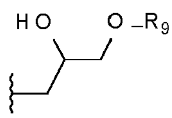 ,

wherein

$R_9$ is $C_1$-$C_{18}$alkyl; or $C_3$-$C_{18}$cycloalkyl; and

$R_7$ is hydrogen; $C_1$-$C_{18}$alkyl; or $C_3$-$C_{12}$cycloalkyl;

wherein at least 2 of $R_2$, $R_3$, $R_4$ and $R_6$ are a radical of formula (1b); and wherein the UV absorber mixture comprises at least 2 different compounds of formula (1).

Preferably, the UV absorber mixture according to the present invention corresponds to compounds of formula (1), wherein

$R_1$ is hydrogen.

UV absorber mixture according to the present invention are preferred, wherein in formula (1)

$R_1$ -O-$R_6$; and
$R_6$ is hydrogen; or a radical of formula

(1b) 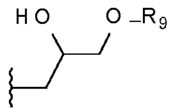 ,

wherein
$R_9$ is $C_1$-$C_{18}$alkyl; or $C_3$-$C_{18}$cycloalkyl.

More preferably, the reaction product according to the present invention corresponds to formula

(1) 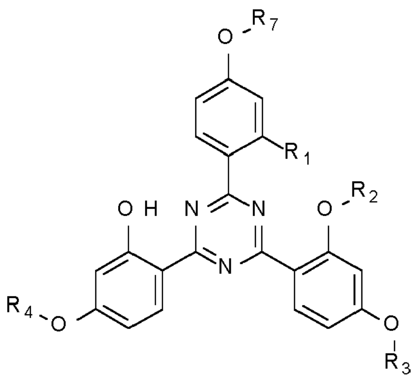 ,

wherein

$R_1$ is hydrogen; or hydroxy;
$R_2$ is hydrogen; or a radical of formula (1a);
$R_3$ and $R_4$ are a radical of formula (1a); and
$R_7$ is hydrogen; $C_1$-$C_{18}$alkyl; or $C_3$-$C_{12}$cycloalkyl.

Preferred are the following UV absorber mixtures:

| | |
|---|---|
| UV-TRIOX-01 | 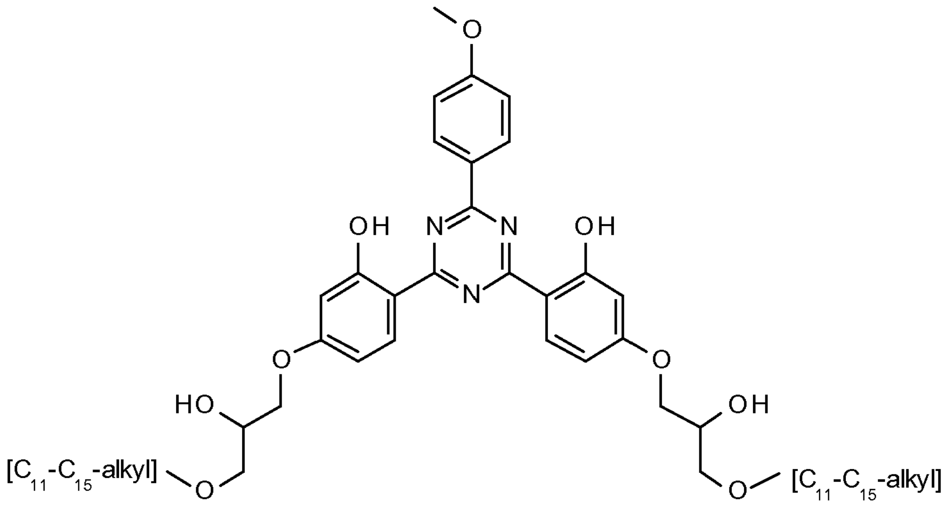 |

(continued)

| | |
|---|---|
| UV-TRIOX-02 | 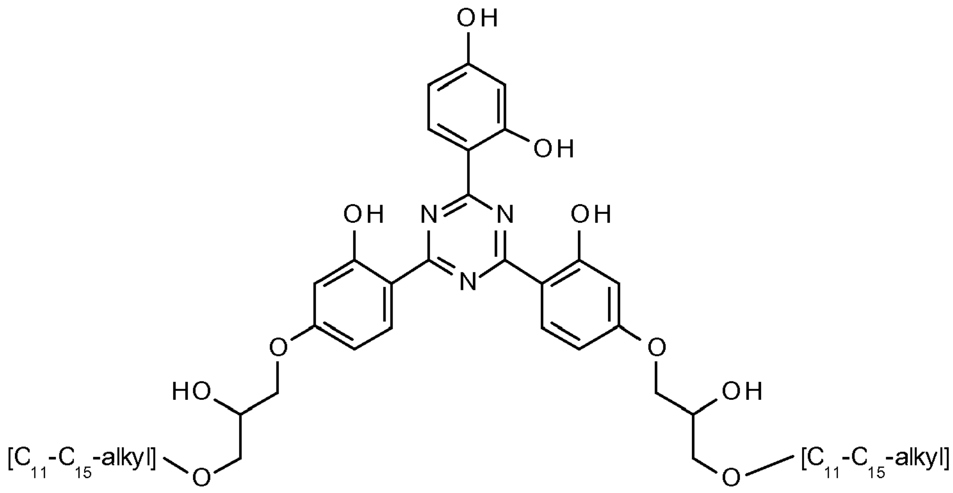 |
| | 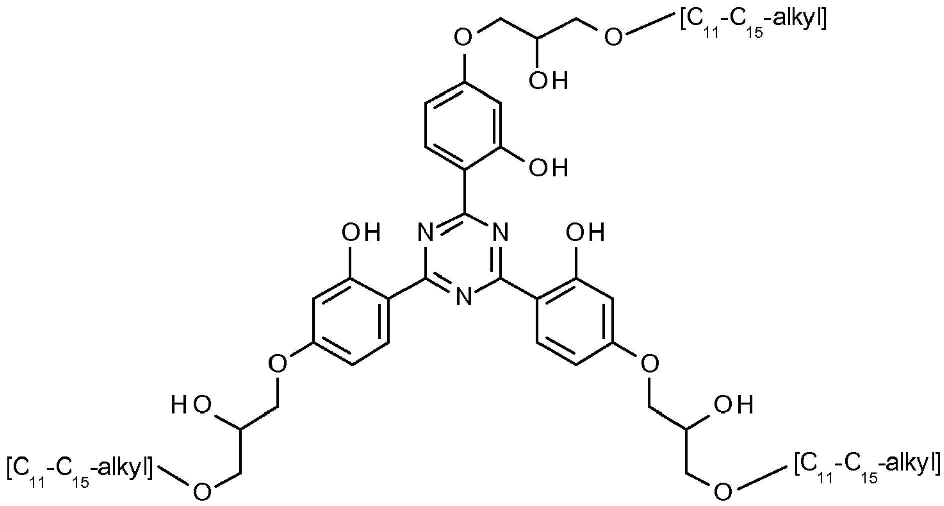 |
| | 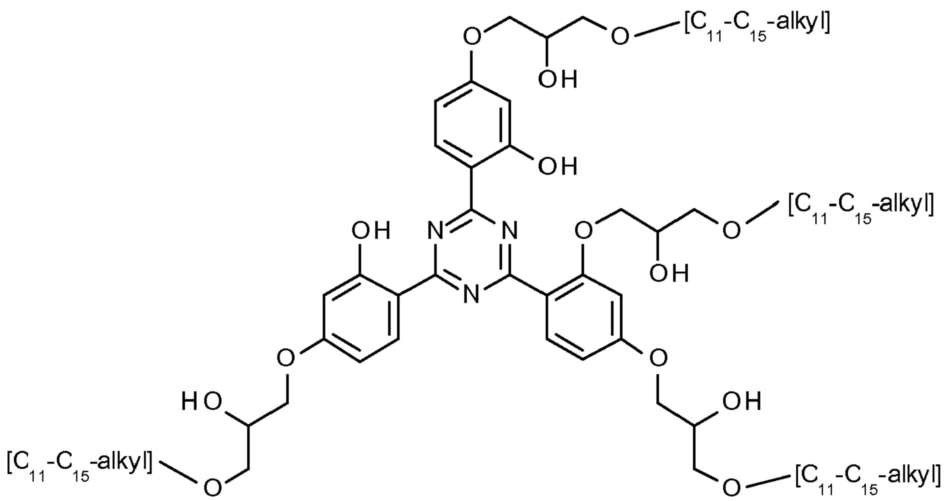 |

The UV absorber mixture of formula (1) is prepared in a manner known per se as known for example from EP 775698. The method for preparation comprises the reaction of alkyl glycidyl ethers of formula (1c) with hydroxyphenyl triazines of formula (4) according to the following reaction scheme:

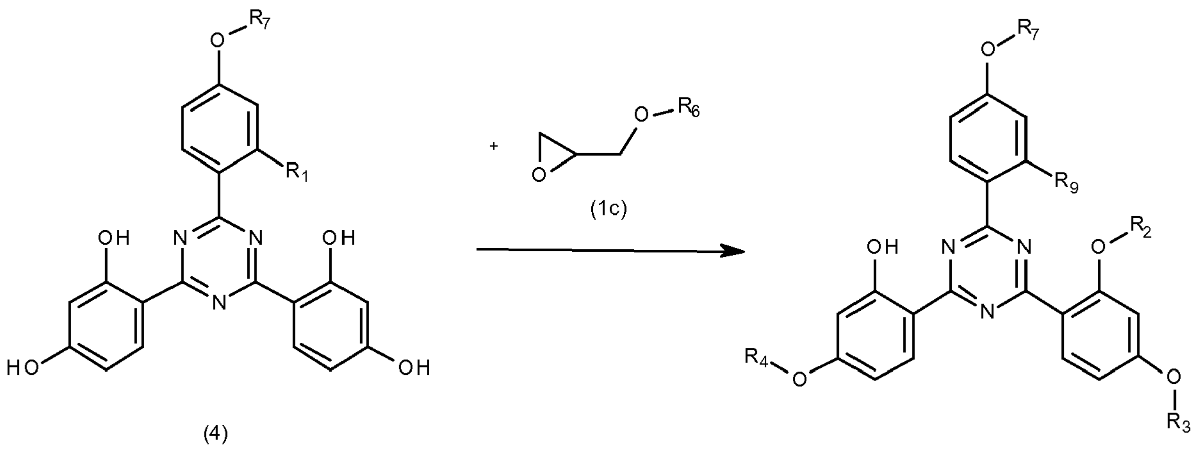

(4) (1c) (1)

wherein

| | |
|---|---|
| $R_1$ | is hydrogen; or hydroxy; |
| $R_2$, $R_3$ and $R_4$, | independently from each other, are hydrogen; or a radical of formula |

(1b) 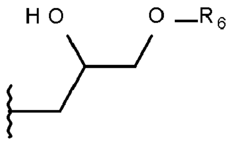 ;

| | |
|---|---|
| $R_6$ | is $C_1$-$C_{18}$alkyl; |
| $R_7$ | is hydrogen; or $C_1$-$C_{18}$alkyl; |
| Rg | is hydrogen; or -O-$R_{10}$; |
| $R_{10}$ | is hydrogen; or a radical of formula (1b); |

wherein at least 2 of the radicals $R_2$, $R_3$, $R_4$ and $R_{10}$ are a radical of formula (1b); and wherein the UV absorber mixture comprises at least 2 different compounds of formula (1).

The reaction may be carried out in non-polar, polar aprotic and polar protic solvents.

Non-polar solvents are for example hexane, xylene, benzene, toluene, 1,4-dioxane, chloroform, diethyl ether or dichloromethane (DCM). Polar aprotic solvents are for example N-methylpyrrolidone, tetrahydrofuran (THF), ethyl acetate (EtOAc), acetone, dimethylformamide (DMF), acetonitrile (MeCN), dimethyl sulfoxide (DMSO) or propylene carbonate (PC). Polar protic solvents are for example formic acid, n-butanol, isopropanol (IPA), nitromethane, ethanol (EtOH), methanol (MeOH), Acetic acid (AcOH), and water.

Preferred solvents in the reaction of the present invention are preferably DMF and mixtures of DMF with aromatic hydrocarbons.

The reaction temperature is carried out from 80-180, preferably from 115-150°C.

Preferably, the UV absorber mixture according to the present invention refers to a UV absorber mixture, which is characterized by the following properties:

- average molecular weight in the range of about 900 to about 5000;
- polydispersity index of about 3 or less;
- solubility of >35% by weight in cosmetic solvents selected from Ethylhexyl Benzoate, C12-15 Alkyl Benzoate, Dibutyl Adipate, Dicaprylyl Carbonate, Dibutyl Sebacate, mixture Undecane and Tridecane (Cetiol Ultimate) and Cocoglycerides;
- solubility of >39% by weight in C12-15 Alkyl Benzoate, Dibutyl Adipate Dicaprylyl Carbonate;

- amorphous at room temperature;
- highly photostable (no degradation detectable after 50 MED irradiation of a 2mg/cm$^2$ film containing 2% of the UV absorber according to the present invention.).

The UV absorber mixture according to the present invention enables sunscreen compositions that provide strong UV radiation protection (high SPF) and minimized skin permeation as well as resistance to washing off with water. Superior sensory profile of cosmetic compositions can be achieved due to high solubility (therefore flexibility in choice of emollients for the formulator). Only one single UV filter composition to achieve sufficient photoprotection for skin Synergy by solubilizing other UV filters, like Tinosorb S. Higher solubility can be achieved in combination compared to single filters.
The UV absorber mixture according to the present invention is especially useful as an active for the protection of UV sensitive organic materials, especially human and animal skin and hair, against the action of UV radiation. Such an UV absorber mixture is preferably suitable as light-protective agent in cosmetic and pharmaceutical applications, especially in sunscreens.
The cosmetic compositions contain, for example, from 0.1 to 50 % by weight, preferably from 0.5 to 20 % by weight, based on the total weight of the composition, of the ultraviolet radiation absorbing polymer composition comprising the polymer compound of formula (3) according to the present invention and a cosmetically tolerable adjuvant.
A typical cosmetic or pharmaceutical composition according to the present invention comprises

(a) from 30 to 90 % by weight, preferably from 50 to 80 % by weight of a continuous water phase;
(b) from 5 to 50 % by weight, preferably from 20 to 50 % by weight of a discontinuous oil phase homogeneously distributed in said water phase and
(c) from 1 to 20 % by weight, preferably from 1 to 5 % by weight of at least one emulsifier selected from

($c_1$) oil-in-water emulsifier;
($c_2$) w/o emulsifier and
($c_3$) w/Si emulsifier

(d) said oil phase comprising 1-70% by weight of the UV absorber mixture as defined claim (1).

Preferably, the cosmetic composition according to the present invention is substantially free of a UV absorber compound other than the UV absorber mixture as defined in claim (1).
Preferably, the UV absorber mixture as defined in claim (1) is present in the cosmetic composition according to the present invention in an amount effective to provide said composition with an SPF of about 10 or greater.
Cosmetic formulations according to the invention are contained in a wide variety of cosmetic preparations, especially the following preparations:

- skin-care preparations, e.g. skin-washing and cleansing preparations in the form of tablet-form or liquid soaps, synthetic detergents or washing pastes,
- bath preparations, e.g. liquid (foam baths, milks, shower preparations) or solid bath preparations, e.g. bath cubes and bath salts;
- skin-care preparations, e.g. skin emulsions, multi-emulsions or skin oils;
- cosmetic personal care preparations, e.g. facial make-up in the form of day creams or powder creams, face powder (loose or pressed), rouge or cream make-up, eye-care preparations, e.g. eye shadow preparations, mascara, eyeliner, eye creams or eye-fix creams; lip-care preparations, e.g. lipsticks, lip gloss, lip contour pencils, nail-care preparations, such as nail varnish, nail varnish removers, nail hardeners or cuticle removers;
- foot-care preparations, e.g. foot baths, foot powders, foot creams or foot balsams, special deodorants and antiperspirants or callus-removing preparations;
- light-protective preparations, such as sun milks, lotions, creams or oils, sunblocks or tropicals, pre-tanning preparations or after-sun preparations;
- skin-tanning preparations, e.g. self-tanning creams;
- depigmenting preparations, e.g. preparations for bleaching the skin or skin-lightening preparations;
- insect-repellents, e.g. insect-repellent oils, lotions, sprays or sticks;
- deodorants, such as deodorant sprays, pump-action sprays, deodorant gels, sticks or roll-ons;
- antiperspirants, e.g. antiperspirant sticks, creams or roll-ons;
- preparations for cleansing and caring for blemished skin, e.g. synthetic detergents (solid or liquid), peeling or scrub preparations or peeling masks;

- hair-removal preparations in chemical form (depilation), e.g. hair-removing powders, liquid hair-removing preparations, cream- or paste-form hair-removing preparations, hair-removing preparations in gel form or aerosol foams;
- shaving preparations, e.g. shaving soap, foaming shaving creams, non-foaming shaving creams, foams and gels, preshave preparations for dry shaving, aftershaves or after-shave lotions;
- fragrance preparations, e.g. fragrances (eau de Cologne, eau de toilette, eau de parfum, parfum de toilette, parfume), parfume oils or parfume creams;
- cosmetic hair-treatment preparations, e.g. hair-washing preparations in the form of shampoos and conditioners, hair-care preparations, e.g. pretreatment preparations, hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colourants, preparations containing self-oxidising dyes, or natural hair colourants, such as henna or camomile.

The final formulations may exist in a wide variety of presentation forms, for example:

- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a powder, a lacquer, a tablet or make-up,
- in the form of a stick,
- in the form of a spray (spray with propellant gas or pump-action spray) or an aerosol,
- in the form of a foam, or
- in the form of a paste.

Important cosmetic compositions for the skin are light-protective preparations, such as sun milks, lotions, creams, oils, sunblocks or tropicals, pretanning preparations or after-sun preparations, also skin-tanning preparations, for example self-tanning creams. Of particular interest are sun protection creams, sun protection lotions, sun protection oils, sun protection milks and sun protection preparations in the form of a spray.

Important cosmetic compositions for the hair are the above-mentioned preparations for hair treatment, especially hair-washing preparations in the form of shampoos, hair conditioners, hair-care preparations, e.g. pretreatment preparations, hair tonics, styling creams, styling gels, pomades, hair rinses, treatment packs, intensive hair treatments, hair-straightening preparations, liquid hair-setting preparations, hair foams and hairsprays. Of special interest are hair-washing preparations in the form of shampoos.

A shampoo has, for example, the following composition:

0.01 to 5 % by weight of a UV absorber mixture according to the present invention,
12.0 % by weight of sodium laureth-2-sulfate,
4.0 % by weight of cocamidopropyl betaine,
3.0 % by weight of sodium chloride, and
water ad 100 %.

Especially the following hair-cosmetic formulations may be used:

$a_1$) spontaneously emulsifying stock formulation, consisting of the UV absorber according to the invention, PEG-6-$C_{10}$oxoalcohol and sorbitan sesquioleate, to which water and any desired quaternary ammonium compound, for example 4 % minkamidopropyl-dimethyl-2-hydroxyethylammonium chloride or Quaternium 80 is added;
$a_2$) spontaneously emulsifying stock formulation consisting of the UV absorber according to the invention, tributyl citrate and PEG-20-sorbitan monooleate, to which water and any desired quaternary ammonium compound, for example 4 % minkamidopropyl-dimethyl-2-hydroxyethylammonium chloride or Quaternium 80 is added;
b) Quat-doped solutions of the UV absorber according to the invention in butyl triglycol and tributyl citrate;
c) mixtures or solutions of the UV absorber according to the invention with n-alkylpyrrolidone.

The cosmetic composition may be, for example a cream, gel, lotion, alcoholic and aqueous/alcoholic solution, emulsion, wax/fat composition, stick preparation, a powder or ointment.

The composition according to the invention, for example the cream, the gel, the lotion, the alcoholic and aqueous/alcoholic solution, the emulsion, the wax/fat composition, the stick preparation, the powder or ointment, may in addition contain, as further adjuvants and additives, mild surfactants, super-fatting agents, pearlescent waxes, consistency regulators, thickeners, polymers, silicone compounds, fats, waxes, stabilisers, biogenic active ingredients, deodorising active ingredients, anti-dandruff agents, film formers, swelling agents, further UV light-protective factors, antioxidants, hydrotropic agents, preservatives, insect repellents, self-tanning agents, solubilisers, perfume oils, colourants, bacteria-inhibiting agents and the like.

Of special importance are light-protective preparations, such as sun milks, lotions, creams, oils, sunblocks or tropicals, pretanning preparations or after-sun preparations, also skin-tanning preparations, for example self-tanning creams. Of particular interest are sun protection creams, sun protection lotions, sun protection oils, sun protection milk and sun protection preparations in the form of a spray.

Of special importance are the above-mentioned preparations for hair treatment, especially hair-washing preparations in the form of shampoos, hair conditioners, hair-care preparations, e.g. pretreatment preparations, hair tonics, styling creams, styling gels, pomades, hair rinses, treatment packs, intensive hair treatments, hair-straightening preparations, liquid hair-setting preparations, hair foams and hairsprays. Of special interest are hair-washing preparations in the form of shampoos.

The cosmetic preparation can be prepared by physically mixing the UV absorber mixture according to the present invention with the adjuvant using conventional methods, for example by simply stirring the individual components together.

As a water-in-oil or oil-in-water emulsion, the cosmetically tolerable adjuvant preferably contains from 5 to 50 % of an oil phase, from 5 to 20 % of an emulsifier and from 30 to 90 % water. The oil phase can comprise any oil suitable for cosmetic formulations, for example one or more hydrocarbon oils, a wax, a natural oil, a silicone oil, a fatty acid ester or a fatty alcohol. Preferred mono- or polyols are ethanol, isopropanol, propylene glycol, hexylene glycol, glycerol and sorbitol.

The cosmetic preparation according to the invention can contain any conventionally usable emulsifier, for example one or more ethoxylated esters of natural derivatives, for example polyethoxylated esters of hydrogenated castor oil, or a silicone oil emulsifier, for example silicone polyol; an unethoxylated or ethoxylated fatty acid soap; an ethoxylated fatty alcohol; an unethoxylated or ethoxylated sorbitan ester; an ethoxylated fatty acid; or an ethoxylated glyceride.

More preferably, the emulsifier ($c_1$), ($c_2$) or ($c_3$) is an anionic or a nonionic emulsifier.

Preferably, the the cosmetic composition according to the present invention is used for minimizing the appearance of dysesthetic feeling of the skin.

In another embodiment of the present invention, the composition according to the present invention comprises additional cosmetic UV absorbers.

Therefore, the present invention relates to a cosmetic composition comprising a UV filter combination of

(a) a UV absorber mixture corresponding to the reaction product of formula (1);

(b) UV filters selected from

($b_1$) an aqueous dispersion of 5,6,5',6'-tetraphenyl-3,3'-(1,4-Phenylene)bis(1,2,4-Triazine) corresponding to the formula

(UV-AD-1) 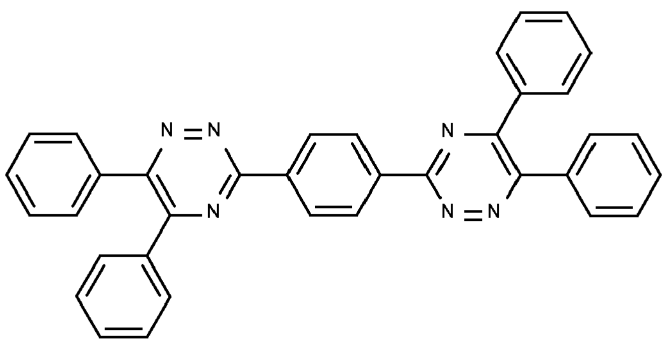

in particulate form; and

($b_2$) Bis-Ethylhexyloxyphenol Methoxyphenyl Triazine;

($b_3$) Butyl Methoxydibenzoylmethane;

($b_4$) Diethylhexyl Butamido Triazone;

($b_5$) Ethylhexyl Triazone;

($b_6$) Diethylamino Hydroxy Benzoyl Hexyl Benzoate;

($b_7$) Ethylhexyl Methoxycinnamate;

($b_8$) Ethylhexyl Salicylate;
($b_9$) Homosalate;
($b_{10}$) Octocrylene;
($b_{11}$) Methylene Bis-Benzotriazolyl Tetramethylbutylphenol;
($b_{12}$) Phenylbenzimidazole Sulfonic Acid;
($b_{13}$) Titanium Dioxide;
($b_{14}$) Tris-Biphenyl Triazine;
($b_{15}$) (2-{4-[2-(4-Diethylamino-2-hydroxy-benzoyl)-benzoyl]-piperazine-1-carbonyl}-phenyl)-(4-diethylamino-2-hydroxy-phenyl)-methanone;
($b_{16}$) BBDAPT; Benzoic acid, 4,4'-[[6-[[3-[1,3,3,3-tetramethyl-1-[(trimethylsilyl)oxy]-1 disiloxanyl]propyl]amino]-1,3,5-triazine-2,4-diyl]diimino]bis-, dibutyl ester;
($b_{17}$) benzylidene malonates;
($b_{18}$) merocyanine derivatives;
($b_{19}$) Bis(butylbenzoate) diaminotriazine aminopropylsiloxane;
($b_{20}$) Bis-Ethylhexyloxyphenol Methoxyphenyl Triazine) encapsulated in a polymer matrix;
($b_{21}$) 2-(2H-Benzotriazol-2-yl)-6-[(2-ethylhexyloxy)methyl]-4-methylphenol;
($b_{22}$) 2-Propenoic acid, 3-(4-methoxyphenyl)-, 2-methylphenyl ester; and
($b_{23}$) Zinc oxide;

wherein said composition contains at least one of the UV filters ($b_1$) - ($b_{23}$); and wherein said composition also contains a pharmaceutically or cosmetically acceptable excipient.

Tinosorb S, Bis-Ethylhexyloxyphenol Methoxyphenyl Triazine encapsulated in a polymer matrix ($b_{20}$) is described in IP.com Journal (2009), 9(1B), 17 (Tinosorb S Aqua, BASF).

2-(2H-Benzotriazol-2-yl)-6-[(2-ethylhexyloxy)methyl]-4-methylphenol ($b_{21}$) corresponds to formula

(UV-AD-2)

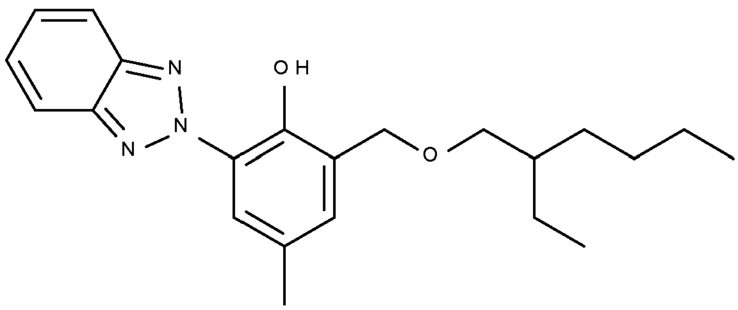

.

2-Propenoic acid, 3-(4-methoxyphenyl)-, 2-methylphenyl ester ($b_{22}$) corresponds to formula

(UV-AD-3)

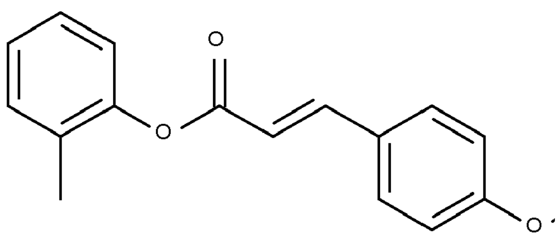

.

Preferably the UV filters ($b_{11}$) Methylene Bis-Benzotriazolyl Tetramethylbutylphenol, ($b_{14}$) Tris-Biphenyl Triazine and ($b_{15}$) (2-{4-[2-(4-Diethylamino-2-hydroxy-benzoyl)-benzoyl]-piperazine-1-carbonyl}-phenyl)-(4-diethylamino-2-hydroxy-phenyl)-methanone are present in the cosmetic or pharmaceutical composition in their micronized state.

The Benzylidene malonates ($b_{17}$) preferably correspond to formula

(UV-AD-4) 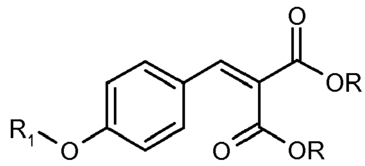 ,

wherein

$R_1$ is methyl; ethyl; propyl; or n-butyl;

if $R_1$ is methyl, then

R is tert. butyl;

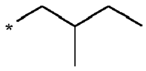 ; 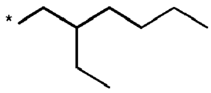 ; 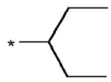 ; 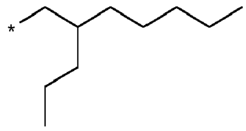 ;

a radical of formula (UV-AD-4a)

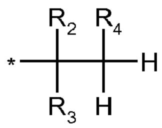 ;

or a radical of formula (UV-AD-4b)

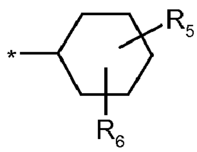 ;

wherein

$R_2$ and $R_3$, independently from each other are hydrogen; or methyl;

$R_4$ is methyl; ethyl; or n-propyl;

$R_5$ and $R_6$ independently from each other are hydrogen; or $C_1$-$C_3$alkyl;

if $R_1$ is ethyl; propyl; or n-butyl, then

R is isopropyl.

Most preferred benzylidene malonate ($b_{17}$) is the compound of formula

(UV-AD-4-01) 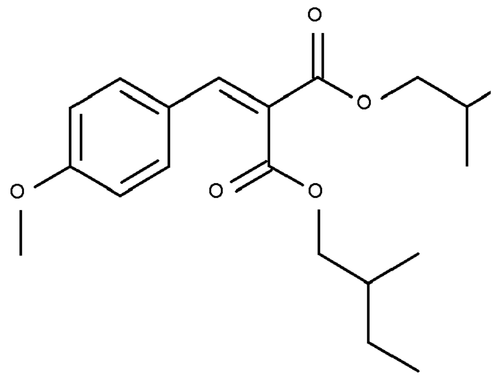

and

(UV-AD-4-02) 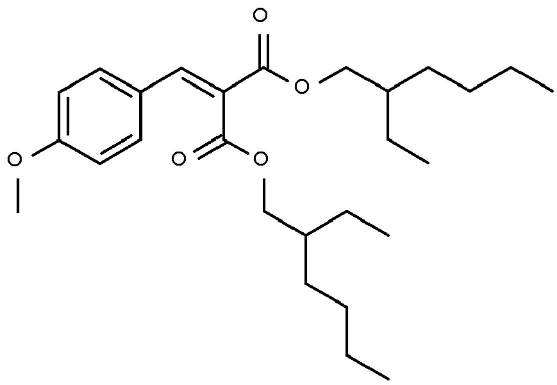 .

The Benzylidene malonates ($b_{17}$) and their use as UV filter in sunscreens are disclosed in detail in WO2010/136360 and WO2011/003774.

The cosmetic composition according to the present invention may comprise, in addition to the UV absorber combination according to the invention, one or more further UV protective agents of the following substance classes: p-aminobenzoic acid derivatives, salicylic acid derivatives, benzophenone derivatives, 3-imidazol-4-yl acrylic acid and esters; benzofuran derivatives, polymeric UV absorbers, camphor derivatives, encapsulated UV absorbers, and 4,4-diphenyl-1,3-butadiene derivatives.

Special preference is given to the UV absorbers indicated in the following Table 1:

| Table 1: Suitable UV filter substances and adjuvants which can be additionally used with the UV absorber mixture according to the present invention | |
|---|---|
| Chemical Name | CAS No. |
| (+/-)-1,7,7-trimethyl-3-[(4-methylphenyl)methylene]bicyclo[2.2.1]heptan-2-one; p-methyl benzylidene camphor | 36861-47-9 |
| 1,7,7-trimethyl-3-(phenylmethylene)bicyclo[2.2.1]heptan-2-one; benzylidene camphor | 15087-24-8 |
| (2-Hydroxy-4-methoxyphenyl)(4-methylphenyl)methanone | 1641-17-4 |
| 2,4-dihydroxybenzophenone | 131-56-6 |
| 2,2',4,4'-tetrahydroxybenzophenone | 131-55-5 |
| 2-Hydroxy-4-methoxy benzophenone; | 131-57-7 |
| 2,2'-dihydroxy-4,4'-dimethoxybenzophenone | 131-54-4 |
| 2,2'-Dihydroxy-4-methoxybenzophenone | 131-53-3 |
| Alpha-(2-oxoborn-3-ylidene)toluene-4-sulphonic acid and its salts (Mexoryl SL) | 56039-58-8 |

(continued)

| Table 1: Suitable UV filter substances and adjuvants which can be additionally used with the UV absorber mixture according to the present invention | |
|---|---|
| Chemical Name | CAS No. |
| Methyl N,N,N-trimethyl-4-[(4,7,7-trimethyl-3-oxobicyclo[2,2,1]hept-2-ylidene)methyl] anilinium sulphate (Mexoryl SO) | 52793-97-2 |
| Isopentyl p-methoxycinnamate; isoamyl methoxy cinnamate | 71617-10-2 |
| Menthyl-o-aminobenzoate | 134-09-8 |
| Menthyl salicylate | 89-46-3 |
| 4- aminobenzoic acid | 150-13-0 |
| Benzoic acid, 4-amino-, ethyl ester, polymer with oxirane | 113010-52-9 |
| 2-Propenamide, N-[[4-[(4,7,7-trimethyl-3-oxobicyclo[2.2.1 ]hept-2-ylidene)methyl] phenyl]methyl]-, homopolymer | 147897-12-9 |
| Triethanolamine salicylate | 2174-16-5 |
| 3,3'-(1,4-phenylenedimethylene)bis[7,7-dimethyl-2-oxo-bicyclo[2.2.1]heptane-1 methanesulfonic acid] (Cibafast H) | 90457-82-2 |
| Zinc oxide (primary particle size 20-100 nm) For example Zinc oxide NDM, Zinc oxide Z-Cote HP1, Nanox Zinc oxide | 1314-13-2 |
| Benzoic acid, 4,4'-[[6-[[4-[[(1,1-dimethylethyl)amino]carbonyl]phenyl]amino]1,3,5-triazine-2,4-diyl]diimino]bis-, bis(2-ethylhexyl)ester; diethylhexyl butamido triazone (Uvasorb HEB) | 154702-15-5 |
| Phenol, 2-(2H-benzotriazol-2-yl)-4-methyl-6-[2-methyl-3-[1,3,3,3-tetramethyl-1-[(trimethylsilyl)oxy]disiloxanyl]propyl]-; drometrizole trisiloxane (Mexoryl XL) | 155633-54-8 |
| Dimethicodiethylbenzalmalonate; Polysilicone 15 (Parsol SLX) | 207574-74-1 |
| Benzenesulfonic acid, 3-(2H-benzotriazol-2-yl)-4-hydroxy-5-(1-methylpropyl)-, monosodium salt (Tinogard HS) | 92484-48-5 |
| 1-Dodecanaminium, N-[3-[[4-(dimethylamino)benzoyl]amino]propyl]N,Ndimethyl-, salt with 4-methylbenzenesulfonic acid (1:1) (Escalol HP610) | 156679-41-3 |
| 1-Propanaminium, N,N,N-trimethyl-3-[(1-oxo-3-phenyl-2-propenyl)amino]-, chloride | 177190-98-6 |
| 1H-Benzimidazole-4,6-disulfonic acid, 2,2'-(1,4-phenylene)bis- | 170864-82-1 |
| 1,3,5-Triazine, 2,4,6-tris(4-methoxyphenyl)- | 7753-12-0 |
| 1,3,5-Triazine, 2,4,6-tris[4-[(2-ethylhexyl)oxy]phenyl]- | 208114-14-1 |
| 1-Propanaminium, 3-[[3-[3-(2H-benzotriazol-2-yl)-5-(1 ,1-dimethylethyl)-4hydroxyphenyl]-1-oxopropyl]amino]-N,N-diethyl-N-methyl-, methyl sulfate (salt) | 340964-15-0 |
| 2-Propenoic acid, 3-(1H-imidazol-4-yl)- | 104-98-3 |
| Benzoic acid, 2-hydroxy-, [4-(1-methylethyl)phenyl]methyl ester | 94134-93-7 |
| 1,2,3-Propanetriol, 1-(4-aminobenzoate) (Glyceryl PABA) | 136-44-7 |
| Benzeneacetic acid, 3,4-dimethoxy-a-oxo- | 4732-70-1 |
| 2-Propenoic acid, 2-cyano-3,3-diphenyl-, ethyl ester | 5232-99-5 |
| Anthralinic acid, p-menth-3-yl ester | 134-09-8 |
| 2,2'-bis(1,4-phenylene)-1H-benzimidazole-4,6-disulphonic acid mono sodium salt or Disodium phenyl dibenzimidazole tetrasulfonate (Neo Heliopan AP) | 349580-12-7 |
| sterols (cholesterol, lanosterol, phytosterols), as described in WO0341675 | |

(continued)

| Table 1: Suitable UV filter substances and adjuvants which can be additionally used with the UV absorber mixture according to the present invention | |
|---|---|
| Chemical Name | CAS No. |
| mycosporines and/or mycosporine-like amino acids as described in WO2002039974, e.g. Helioguard 365 from Milbelle AG, isolated mycosporine like amino acids from the red alga porphyra umbilicalis (INCI: Porphyra Umbilicalis) that are encapsulated into liposomes) | |
| alpha-lipoic-acid as described in DE 10229995 | |
| synthetic organic polymers as described in EP 1 371 358, [0033]-[0041] | |
| phyllosilicates as described in EP 1371357 [0034]-[0037] | |
| silica compounds as described in EP1371356, [0033]-[0041] | |
| inorganic particles as described in DE10138496 [0043]-[0055] | |
| latex particles as described in DE10138496 [0027]-[0040] | |
| 1H-Benzimidazole-4,6-disulfonic acid, 2,2'-(1,4-phenylene)bis-, disodium salt; Bisimidazylate (Neo Heliopan APC) | 180898-37-7 |
| Di-2-ethylhexyl-3,5-dimethoxy-4-hydroxy-benzalmalonate (Oxyn ex ST, EMD Chemicals, as described in US 20040247536) | |
| Z-COTE® MAX: Zinc Oxide (and) Diphenyl Capryl Methicone | |
| Z-COTE HP1: Zinc Oxide (and) Triethoxycaprylylsilane | |
| 1,3,5-Triazine-2,4,6-triamine, N2,N4-bis[4-[5-(1,1-dimethylpropyl)-2-benzoxazolyl] phenyl]-N6-(2-ethylhexyl)-(Uvasorb K2A) | 288254-16-0 |
| 1,1-[(2,2'-Dimethylpropoxy)carbonyl]-4,4-diphenyl-1,3-butadiene | 363602-15-7 |
| UV filter capsules containing an organic sunscreen as described in DE102007035567 or WO 2009012871 | |

The UV absorber mixture according to the present invention can preferably used for the enhancement of the solubility of other UV absorbers present in the cosmetic formulation according to the present invention.

The cosmetic composition according to the present invention can be prepared by physically mixing the ultraviolet radiation absorbing polymer composition with the adjuvant using customary methods, for example by simply stirring together the individual components, especially by making use of the dissolution properties of already known cosmetic UV absorbers, for example Ethylhexyl Methoxycinnamate. The UV absorbers can be used, for example, without further treatment.

In addition to other properties, the cosmetic composition according to the present invention can be used as a radical scavenger by reducing significantly the number of UV- induced free radicals in skin when applied in a suitable cosmetic carrier.

If the compositions according to the present invention represent water- and oil-containing emulsions (e.g. W/O, O/W, O/W/O and W/O/W emulsions or microemulsions) they contain, for example, from 0.1 to 70 % by weight, preferably from 0.1 to 30 % by weight and especially from 0.5 to 10 % by weight, based on the total weight of the composition, of the UV absorber mixture according to the present invention, from 1 to 60 % by weight, especially from 5 to 50 % by weight and preferably from 10 to 35 % by weight, based on the total weight of the composition, of at least one oil component, from 0 to 30 % by weight, especially from 1 to 30 % by weight and preferably from 4 to 20 % by weight, based on the total weight of the composition, of at least one emulsifier, from 10 to 90 % by weight, especially from 30 to 90 % by weight, based on the total weight of the composition, of water, and from 0 to 88.9 % by weight, especially from 1 to 50 % by weight, of further cosmetically tolerable adjuvants.

Suitable oil components of oil-containing compositions (e.g. oils, W/O, O/W, O/W/O and W/O/W emulsions or microemulsions) are for example Guerbet alcohols based on fatty alcohols having from 6 to 18, preferably from 8 to 10, carbon atoms, esters of linear $C_6$-$C_{24}$ fatty acids with linear $C_3$-$C_{24}$ alcohols, esters of branched $C_6$-$C_{13}$carboxylic acids with linear $C_6$-$C_{24}$ fatty alcohols, esters of linear $C_6$-$C_{24}$ fatty acids with branched alcohols, especially 2-ethylhexanol, esters of hydroxycarboxylic acids with linear or branched $C_6$-$C_{22}$ fatty alcohols, especially dioctyl malates, esters of

linear and/or branched fatty acids with polyhydric alcohols (for example propylene glycol, dimer diol or trimer triol) and/or Guerbet alcohols, triglycerides based on $C_6$-$C_{10}$ fatty acids, liquid mono-/di-/tri-glyceride mixtures based on $C_6$-$C_{18}$ fatty acids, esters of $C_6$-$C_{24}$ fatty alcohols and/or Guerbet alcohols with aromatic carboxylic acids, especially benzoic acid, esters of $C_2$-$C_{12}$dicarboxylic acids with linear or branched alcohols having from 1 to 22 carbon atoms or polyols having from 2 to 10 carbon atoms and from 2 to 6 hydroxy groups, vegetable oils (such as sunflower oil, olive oil, soybean oil, rapeseed oil, almond oil, jojoba oil, orange oil, wheat germ oil, peach kernel oil and the liquid components of coconut oil), branched primary alcohols, substituted cyclohexanes, linear and branched $C_6$-$C_{22}$ fatty alcohol carbonates, Guerbet carbonates, esters of benzoic acid with linear and/or branched $C_6$-$C_{22}$alcohols (e.g. Finsolv® TN), linear or branched, symmetric or asymmetric di-alkyl ethers having a total of from 12 to 36 carbon atoms, especially from 12 to 24 carbon atoms, for example di-n-octyl ether, di-n-decyl ether, di-n-nonyl ether, di-n-undecyl ether, di-n-dodecyl ether, n-hexyl n-octyl ether, n-octyl n-decyl ether, n-decyl n-undecyl ether, n-undecyl n-dodecyl ether, n-hexyl n-undecyl ether, di-tert-butyl ether, diisopentyl ether, di-3-ethyldecyl ether, tert-butyl n-octyl ether, isopentyl n-octyl ether and 2-methyl pentyl-n-octyl ether; ring-opening products of epoxidised fatty acid esters with polyols, silicone oils and/or aliphatic or naphthenic hydrocarbons. Also of importance are monoesters of fatty acids with alcohols having from 3 to 24 carbon atoms. That group of substances comprises the esterification products of fatty acids having from 8 to 24 carbon atoms, for example caproic acid, caprylic acid, 2-ethylhexanoic acid, capric acid, lauric acid, isotridecanoic acid, myristic acid, palmitic acid, palmitoleic acid, stearic acid, isostearic acid, oleic acid, elaidic acid, petroselinic acid, linoleic acid, linolenic acid, elaeostearic acid, arachidic acid, gadoleic acid, behenic acid and erucic acid and technical-grade mixtures thereof (obtained, for example, in the pressure removal of natural fats and oils, in the reduction of aldehydes from Roelen's oxosynthesis or in the dimerisation of unsaturated fatty acids) with alcohols, for example isopropyl alcohol, caproic alcohol, capryl alcohol, 2-ethylhexyl alcohol, capric alcohol, lauryl alcohol, isotridecyl alcohol, myristyl alcohol, cetyl alcohol, palmoleyl alcohol, stearyl alcohol, isostearyl alcohol, oleyl alcohol, elaidyl alcohol, petroselinyl alcohol, linoyl alcohol, linolenyl alcohol, elaeostearyl alcohol, arachidyl alcohol, gadoleyl alcohol, behenyl alcohol, erucyl alcohol and brassidyl alcohol and technical-grade mixtures thereof (obtained, for example, in the high-pressure hydrogenation of technical-grade methyl esters based on fats and oils or aldehydes from Roelen's oxosynthesis and as monomer fractions in the dimerisation of unsaturated fatty alcohols). Of special importance are isopropyl myristate, isononanoic acid $C_{16}$-$C_{18}$alkyl esters, stearic acid 2-ethylhexyl ester, cetyl oleate, glycerol tricaprylate, coconut fatty alcohol caprinate/caprylate and n-butyl stearate. Further oil components that can be used are dicarboxylic acid esters, such as di-n-butyl adipate, di(2-ethylhexyl) adipate, di(2-ethylhexyl) succinate and diisotridecyl acetate, and also diol esters, such as ethylene glycol dioleate, ethylene glycol diisotridecanoate, propylene glycol di(2-ethylhexanoate), propylene glycol diisostearate, propylene glycol dipelargonate, butanediol diisostearate and neopentyl glycol di-caprylate. Preferred mono- or poly-ols are ethanol, isopropanol, propylene glycol, hexylene glycol, glycerol and sorbitol. It is also possible to use di- and/or trivalent metal salts (alkaline earth metal, $Al^{3+}$ *inter alia*) of one or more alkyl carboxylic acids.

The oil components can be used in an amount of, for example, from 1 to 60 % by weight, especially from 5 to 50 % by weight and preferably from 10 to 35 % by weight, based on the total weight of the composition.

Any conventionally emulsifier can be used for the cosmetic compositions according to the present invention.

Suitable emulsifiers are for example, non-ionic surfactants from the following groups:

- addition products of from 2 to 30 mol of ethylene oxide and/or from 0 to 5 mol of propylene oxide with linear fatty alcohols having from 8 to 22 carbon atoms, with fatty acids having from 12 to 22 carbon atoms and with alkylphenols having from 8 to 15 carbon atoms in the alkyl group, for example ceteareth-20 or ceteareth-12;
- $C_{12}$-$C_{22}$ fatty acid mono- and di-esters of addition products of from 1 to 30 mol of ethylene oxide with polyols having from 3 to 6 carbon atoms, especially with glycerol;
- glycerol mono- and di-esters and sorbitan mono- and di-esters of saturated and unsaturated fatty acids having from 6 to 22 carbon atoms and ethylene oxide addition products thereof, for example glyceryl stearates, glyceryl isostearates, glyceryl oleates, sorbitan oleates or sorbitan sesquioleates;
- $C_8$-$C_{22}$alkyl-mono- and -oligo-glycosides and ethoxylated analogues thereof, degrees of oligomerisation of from 1.1 to 5, especially from 1.2 to 1.4, being preferred, and glucose being preferred as the sugar component;
- addition products of from 2 to 60 mol, especially from 15 to 60 mol, of ethylene oxide with castor oil and/or hydrogenated castor oil;
- polyol esters and especially polyglycerol esters, for example diisostearoyl polyglyceryl-3-diisostearates, polyglyceryl-3-diisostearates, triglyceryl diisostearates, polyglyceryl-2-sesquiisostearates or polyglyceryl dimerates. Mixtures of compounds from a plurality of those substance classes are also suitable;
- partial esters based on linear, branched, unsaturated or saturated $C_6$-$C_{22}$ fatty acids, ricinoleic acid and also 12-hydroxystearic acid and on glycerol, polyglycerol, pentaerythritol, dipentaerythritol, sugar alcohols (e.g. sorbitol), alkyl glucosides (e.g. methyl glucoside, butyl glucoside, lauryl glucoside) and also polyglucosides (e.g. cellulose), for example polyglyceryl-2-dihydroxystearates or polyglyceryl-2-diricinoleates;
- mono-, di- and tri-alkylphosphates and also mono-, di- and/or tri-PEG-alkylphosphates and salts thereof;

- wool wax alcohols;
- one or more ethoxylated esters of natural derivatives, for example polyethoxylated esters of hydrogenated castor oil;
- silicone oil emulsifiers, for example silicone polyol;
- polysiloxane/polyalkyl/polyether copolymers and corresponding derivatives, for example cetyl dimethicone copolyol;
- mixed esters of pentaerythritol, fatty acids, citric acid and fatty alcohol (see DE-A-1 165 574) and/or mixed esters of fatty acids having from 6 to 22 carbon atoms, methylglucose and polyols, preferably glycerol or polyglycerol, for example polyglyceryl-3-glucose distearates, polyglyceryl-3-glucose dioleates, methyl glucose dioleates or dicocoyl pentaerythryl distearyl citrates; and also
- polyalkylene glycols.

The addition products of ethylene oxide and/or of propylene oxide with fatty alcohols, fatty acids, alkylphenols, glycerol mono- and di-esters and also sorbitan mono- and di-esters of fatty acids, or with castor oil, are known, commercially available products. They are usually homologue mixtures, the average degree of alkoxylation of which corresponds to the ratio of the amounts of ethylene oxide and/or propylene oxide and substrate with which the addition reaction is carried out. $C_{12}$-$C_{18}$ fatty acid mono- and di-esters of addition products of ethylene oxide with glycerol are known, for example, from DE-A-2 024 051 as fat-restoring substances for cosmetic preparations.

$C_8$-$C_{18}$Alkyl-mono- and -oligo-glycosides, their preparation and their use are known from the prior art. They are prepared especially by reacting glucose or oligosaccharides with primary alcohols having from 8 to 18 carbon atoms. Suitable glycoside radicals include monoglycosides in which a cyclic sugar radical is glycosidically bonded to the fatty alcohol and also oligomeric glycosides having a degree of oligomerisation of up to preferably about 8. The degree of oligomerisation is a statistical average value based on a homologue distribution customary for such technical-grade products.

It is also possible to use zwitterionic surfactants as emulsifiers. The term "zwitterionic surfactants" denotes especially surface-active compounds that carry at least one quaternary ammonium group and at least one carboxylate and/or sulfonate group in the molecule. Zwitterionic surfactants that are especially suitable are the so-called betaines, such as N-alkyl-N,N-dimethylammonium glycinates, for example cocoalkyldimethylammonium glycinate, N-acylaminopropyl-N,N-dimethylammonium glycinates, for example cocoacylaminopropyldimethylammonium glycinate, and 2-alkyl-3-carboxymethyl-3-hydroxyethylimidazolines each having from 8 to 18 carbon atoms in the alkyl or acyl group and also cocoacylaminoethylhydroxyethylcarboxymethylglycinate. Special preference is given to the fatty acid amide derivative known by the CTFA name cocamidopropyl betaine. Likewise suitable as emulsifiers are ampholytic surfactants. Ampholytic surfactants are to be understood as meaning especially those which, in addition to containing a $C_8$-$C_{18}$-alkyl or -acyl group, contain at least one free amino group and at least one -COOH or -$SO_3$H group in the molecule and are capable of forming internal salts. Examples of suitable ampholytic surfactants include N-alkylglycines, N-alkylpropionic acids, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids and alkylaminoacetic acids, each having approximately from 8 to 18 carbon atoms in the alkyl group.

Ampholytic surfactants to which special preference is given are N-cocoalkylamino-propionate, cocoacylaminoethylaminopropionate and $C_{12}$-$C_{18}$acylsarcosine. In addition to the ampholytic emulsifiers there also come into consideration quaternary emulsifiers, special preference is given to those of the esterquat type, preferably methyl-quaternised di-fatty acid triethanolamine ester salts.

Non-ionic emulsifiers are preferred, preferably ethoxylated fatty alcohols having from 8 to 22 carbon atoms and from 4 to 30 EO units.

The emulsifiers may be used in an amount of, for example, from 1 to 30 % by weight, especially from 4 to 20 % by weight and preferably from 5 to 10 % by weight, based on the total weight of the composition. It is, however, also possible in principle to dispense with the use of emulsifiers.

The compositions according to the invention, for example creams, gels, lotions, alcoholic and aqueous/alcoholic solutions, emulsions, wax/fat compositions, stick preparations, powders or ointments, may in addition contain, as further adjuvants and additives, mild surfactants, super-fatting agents, pearlescent waxes, consistency regulators, thickeners, polymers, silicone compounds, fats, waxes, stabilisers, biogenic active ingredients, deodorising active ingredients, antidandruff agents, film formers, swelling agents, antioxidants, hydrotropic agents, preservatives, insect repellents, self-tanning agents, solubilizers, perfume oils, colorants, bacteria-inhibiting agents and the like.

Substances suitable for use as super-fatting agents are, for example, lanolin and lecithin and also polyethoxylated or acrylated lanolin and lecithin derivatives, polyol fatty acid esters, monoglycerides and fatty acid alkanolamides, the latter simultaneously acting as foam stabilisers.

Examples of suitable mild surfactants, that is to say surfactants especially well tolerated by the skin, include fatty alcohol polyglycol ether sulfates, monoglyceride sulfates, mono- and/or di-alkyl sulfosuccinates, fatty acid isethionates, fatty acid sarcosinates, fatty acid taurides, fatty acid glutamates, $\alpha$-olefin sulfonates, ether carboxylic acids, alkyl oligoglucosides, fatty acid glucamides, alkylamidobetaines and/or protein fatty acid condensation products, the latter

preferably being based on wheat proteins.

Suitable pearlescent are for example: alkylene glycol esters, especially ethylene glycol distearate; fatty acid alkanolamides, especially coco fatty acid diethanolamide; partial glycerides, especially stearic acid monoglyceride; esters of polyvalent, unsubstituted or hydroxy-substituted carboxylic acids with fatty alcohols having from 6 to 22 carbon atoms, especially long-chained esters of tartaric acid; fatty substances, for example fatty alcohols, fatty ketones, fatty aldehydes, fatty ethers and fatty carbonates, which in total have at least 24 carbon atoms, especially laurone and distearyl ether; fatty acids, such as stearic acid, hydroxystearic acid or behenic acid, ring-opening products of olefin epoxides having from 12 to 22 carbon atoms with fatty alcohols having from 12 to 22 carbon atoms and/or polyols having from 2 to 15 carbon atoms and from 2 to 10 hydroxy groups, and mixtures thereof.

Suitable consistency regulators are especially fatty alcohols or hydroxy fatty alcohols having from 12 to 22 carbon atoms and preferably from 16 to 18 carbon atoms, and in addition partial glycerides, fatty acids and hydroxy fatty acids. Preference is given to a combination of such substances with alkyl-oligoglucosides and/or fatty acid N-methylglucamides of identical chain length and/or polyglycerol poly-12-hydroxystearates. Suitable thickeners include, for example, Aerosil types (hydrophilic silicic acids), polysaccharides, especially xanthan gum, guar-guar, agar-agar, alginates and Tyloses, carboxymethyl cellulose and hydroxymethyl cellulose, also relatively high molecular weight polyethylene glycol mono- and di-esters of fatty acids, polyacrylates (e.g. Carbopol® from Goodrich or Synthalen® from Sigma), polyacrylamides, polyvinyl alcohol and polyvinylpyrrolidone, surfactants, for example ethoxylated fatty acid glycerides, esters of fatty acids with polyols, for example pentaerythritol or trimethylolpropane, fatty alcohol ethoxylates with restricted homologue distribution and alkyl-oligoglucosides as well as electrolytes, such as sodium chloride or ammonium chloride.

Suitable cationic polymers are, for example, cationic cellulose derivatives, for example a quarternised hydroxymethyl cellulose obtainable under the name Polymer JR 400® from Amerchol, cationic starch, copolymers of diallylammonium salts and acrylamides, quaternised vinylpyrrolidone/vinyl imidazole polymers, for example Luviquat® (BASF), condensation products of polyglycols and amines, quaternised collagen polypeptides, for example lauryldimonium hydroxypropyl hydrolyzed collagen (Lamequat®L/Grünau), quarternised wheat polypeptides, polyethyleneimine, cationic silicone polymers, for example amidomethicones, copolymers of adipic acid and dimethylaminohydroxypropyldiethylenetriamine (Cartaretin®/Sandoz), copolymers of acrylic acid with dimethyldiallylammonium chloride (Merquat® 550/Chemviron), polyaminopolyamides, as described, for example, in FR-A-2 252 840, and the crosslinked water-soluble polymers thereof, cationic chitin derivatives, for example quaternised chitosan, optionally distributed as microcrystals; condensation products of dihaloalkyls, for example dibromobutane, with bisdialkylamines, for example bisdimethylamino-1,3-propane, cationic guar gum, for example Jaguar® C-17, Jaguar® C-16 from Celanese, quaternised ammonium salt polymers, for example Mirapol® A-15, Mirapol® AD-1, Mirapol® AZ-1 from Miranol.

Suitable anionic, zwitterionic, amphoteric and non-ionic polymers are for example, vinyl acetate/crotonic acid copolymers, vinylpyrrolidone/vinyl acrylate copolymers, vinyl acetate/butyl maleate/isobornyl acrylate copolymers, methyl vinyl ether/maleic anhydride copolymers and esters thereof, uncrosslinked polyacrylic acids and polyacrylic acids crosslinked with polyols, acrylamidopropyltrimethylammonium chloride/acrylate copolymers, octyl acrylamide/methyl methacrylate/tert-butylaminoethyl methacrylate/2-hydroxypropyl methacrylate copolymers, polyvinylpyrrolidone, vinylpyrrolidone/vinyl acetate copolymers, vinylpyrrolidone/dimethylaminoethyl methacrylate/vinyl caprolactam terpolymers and also optionally derivatised cellulose ethers and silicones.

Suitable silicone compounds are, for example, dimethylpolysiloxanes, methylphenylpolysiloxanes, cyclic silicones, and also amino-, fatty acid-, alcohol-, polyether-, epoxy-, fluorine-, glycoside- and/or alkyl-modified silicone compounds, which at room temperature may be in either liquid or resinous form. Also suitable are simethicones, which are mixtures of dimethicones having an average chain length of from 200 to 300 dimethylsiloxane units with hydrogenated silicates. A detailed survey by Todd et al. of suitable volatile silicones may in addition be found in Cosm. Toil. 91, 27 (1976).

Typical examples of fats are glycerides, examples for waxes are inter *inter alia* beeswax, carnauba wax, candelilla wax, montan wax, paraffin wax, hydrogenated castor oils and fatty acid esters or microwaxes solid at room temperature optionally in combination with hydrophilic waxes, e.g. cetylstearyl alcohol or partial glycerides. Metal salts of fatty acids, for example magnesium, aluminium and/or zinc stearate or ricinoleate, may be used as stabilizers.

Biogenic active ingredients are for example, tocopherol, tocopherol acetate, tocopherol palmitate, ascorbic acid, deoxyribonucleic acid, retinol, bisabolol, allantoin, phytantriol, panthenol, AHA acids, amino acids, ceramides, pseudoceramides, essential oils, plant extracts and vitamin complexes.

Suitable deodorizing active ingredients are for example, antiperspirants like aluminium chlorohydrates (see J. Soc. Cosm. Chem. 24, 281 (1973)). Aluminium chlorohydrate corresponding to formula $Al_2(OH)_5Cl \times 2.5\ H_2O$, known and commercially available under the trade mark Locron® of Hoechst AG, Frankfurt (FRG), is especially preferred (see J. Pharm. Pharmacol. 26, 531 (1975)). Beside the chlorohydrates, it is also possible to use aluminium hydroxy-acetates and acidic aluminium/zirconium salts. Esterase inhibitors may be added as further deodorising active ingredients. Such inhibitors are preferably trialkyl citrates, such as trimethyl citrate, tripropyl citrate, triisopropyl citrate, tributyl citrate and especially triethyl citrate (Hydagen® CAT, Henkel KGaA, Düsseldorf/FRG), which inhibit enzyme activity and hence reduce odour formation. Further suitable esterase inhibitors are sterol sulfates or phosphates, for example lanosterol,

cholesterol, campesterol, stigmasterol and sitosterol sulfate or phosphate, dicarboxylic acids and esters thereof, for example glutaric acid, glutaric acid monoethyl ester, glutaric acid diethyl ester, adipic acid, adipic acid monoethyl ester, adipic acid diethyl ester, malonic acid and malonic acid diethyl ester and hydroxycarboxylic acids and esters thereof, for example citric acid, malic acid, tartaric acid or tartaric acid diethyl ester. Antibacterial active ingredients that influence the microbial flora and kill, or inhibit the growth of, sweat-decomposing bacteria can likewise be present in the preparations (especially in stick preparations). Examples include chitosan, phenoxyethanol and chlorhexidine gluconate. 5-Chloro-2-(2,4-dichlorophenoxy)-phenol (Irgasan®, BASF has also proved especially effective.

Suitable anti-dandruff agents are for example, climbazole, octopirox and zinc pyrithione. Customary film formers include, for example, chitosan, microcrystalline chitosan, quaternised chitosan, polyvinylpyrrolidone, vinylpyrrolidone/vinyl acetate copolymers, polymers of quaternary cellulose derivatives containing a high proportion of acrylic acid, collagen, hyaluronic acid and salts thereof and similar compounds. Suitable swelling agents for aqueous phases are montmorillonites, clay mineral substances, Pemulen and also alkyl-modified types of Carbopol (Goodrich). Further suitable polymers and swelling agents can be found in the review by R. Lochhead in Cosm. Toil. 108, 95 (1993).

In addition to the primary UV absorbers it is also possible to use secondary light-protective substances of the antioxidant type which interrupt the photochemical reaction chain triggered when UV radiation penetrates the skin or hair. Typical examples of such antioxidants are amino acids (e.g. glycine, histidine, tyrosine, tryptophan) and derivatives thereof, imidazoles (e.g. urocanic acid) and derivatives thereof, peptides, such as D,L-carnosine, D-carnosine, L-carnosine and derivatives thereof (e.g. anserine), carotinoids, carotenes (e.g. $\alpha$-carotene, $\beta$-carotene, lycopene) and derivatives thereof, chlorogenic acid and derivatives thereof, lipoic acid and derivatives thereof (e.g. dihydrolipoic acid), aurothioglycose, propylthiouracil and other thiols (e.g. thioredoxin, glutathione, cysteine, cystine, cystamine and the glycosyl, N-acetyl, methyl, ethyl, propyl, amyl, butyl, lauryl, palmitoyl, oleyl, $\gamma$-linoleyl, cholesteryl and glyceryl esters thereof) and also salts thereof, dilauryl thiodipropionate, distearyl thiodipropionate, thiodipropionic acid and derivatives thereof (esters, ethers, peptides, lipids, nucleotides, nucleosides and salts) and also sulfoximine compounds (e.g. buthionine sulfoximines, homo-cysteine sulfoximine, buthionine sulfones, penta-, hexa-, hepta-thionine sulfoximine) in very small tolerable amounts (e.g. from pmol to $\mu$mol/kg), also (metal) chelating agents (e.g. $\alpha$-hydroxy fatty acids, palmitic acid, phytic acid, lactoferrin), $\alpha$-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. $\gamma$-linolenic acid, linoleic acid, oleic acid), folic acid and derivatives thereof, ubiquinone and ubiquinol and derivatives thereof, vitamin C and derivatives (e.g. ascorbyl palmitate, magnesium ascorbyl phosphate, ascorbyl acetate), tocopherols and derivatives (e.g. vitamin E acetate), vitamin A and derivatives (e.g. vitamin A palmitate) and also coniferyl benzoate of benzoin resin, rutinic acid and derivatives thereof, $\alpha$-glycosylrutin, ferulic acid, furfurylidene glucitol, carnosine, butyl hydroxytoluene, butyl hydroxyanisole, resinous nordihydroguaiaretic acid, nordihydroguaiaretic acid, trihydroxybutyrophenone, uric acid and derivatives thereof, mannose and derivatives thereof, superoxide dismutase, N-[3-(3,5-di-tert-butyl-4-hydroxyphenyl)propionyl]sulfanilic acid (and salts thereof, for example the sodium salts), zinc and derivatives thereof (e.g. ZnO, $ZnSO_4$), selenium and derivatives thereof (e.g. selenium methionine), stilbene and derivatives thereof (e.g. stilbene oxide, trans-stilbene oxide) and the derivatives suitable according to the invention (salts, esters, ethers, sugars, nucleotides, nucleosides, peptides and lipids) of those mentioned active ingredients. HALS (="Hindered Amine Light Stabilizers") compounds may also be mentioned. The amount of antioxidants present is usually from 0.001 to 30 % by weight, preferably from 0.01 to 3 % by weight, based on the weight of the cosmetic composition according to the present invention.

For improvement of the flow behavior it is also possible to employ hydrotropic agents, for example ethanol, isopropyl alcohol or polyols. Suitable polyols for that purpose comprise preferably from 2 to 15 carbon atoms and at least two hydroxy groups.

The polyols may also contain further functional groups, especially amino groups, and/or may be modified with nitrogen. Typical examples are as follows:

- glycerol;
- alkylene glycols, for example ethylene glycol, diethylene glycol, propylene glycol, butylene glycol, hexylene glycol and also polyethylene glycols having an average molecular weight of from 100 to 1000 dalton;
- technical oligoglycerol mixtures having an intrinsic degree of condensation of from 1.5 to 10, for example technical diglycerol mixtures having a diglycerol content of from 40 to 50 % by weight;
- methylol compounds, such as, especially, trimethylolethane, trimethylolpropane, trimethyl-olbutane, pentaerythritol and dipentaerythritol;
- lower alkyl-glucosides, especially those having from 1 to 8 carbon atoms in the alkyl radical, for example methyl and butyl glucoside;
- sugar alcohols having from 5 to 12 carbon atoms, for example sorbitol or mannitol;
- sugars having from 5 to 12 carbon atoms, for example glucose or saccharose;
- amino sugars, for example glucamine;

- dialcohol amines, such as diethanolamine or 2-amino-1,3-propanediol.

Suitable preservatives include, for example, phenoxyethanol, formaldehyde solution, Parabens, pentanediol or sorbic acid and the further substance classes listed in Schedule 6, Parts A and B of the Cosmetics Regulations.

Suitable perfume oils are mixtures of natural and/or synthetic aromatic substances. Representatives of natural aromatic substances are, for example, extracts from blossom (lilies, lavender, roses, jasmine, neroli, ylang-ylang), from stems and leaves (geranium, patchouli, petitgrain), from fruit (aniseed, coriander, carraway, juniper), from fruit peel (bergamot, lemons, oranges), from roots (mace, angelica, celery, cardamom, costus, iris, calmus), from wood (pinewood, sandalwood, guaiacum wood, cedarwood, rosewood), from herbs and grasses (tarragon, lemon grass, sage, thyme), from needles and twigs (spruce, pine, Scots pine, mountain pine), from resins and balsams (galbanum, elemi, benzoin, myrrh, olibanum, opoponax). Animal raw materials also come into consideration, for example civet and castoreum. Typical synthetic aromatic substances are, for example, products of the ester, ether, aldehyde, ketone, alcohol or hydrocarbon type.

Aromatic substance compounds of the ester type are, for example, benzyl acetate, phenoxyethyl isobutyrate, p-tert-butylcyclohexyl acetate, linalyl acetate, dimethyl-benzylcarbinyl acetate, phenylethyl acetate, linalyl benzoate, benzyl formate, ethylmethylphenyl glycinate, allyl-cyclohexyl propionate, styrallyl propionate and benzyl salicylate. The ethers include, for example, benzyl ethyl ether; the aldehydes include, for example, the linear alkanals having from 8 to 18 hydrocarbon atoms, citral, citronellal, citronellyl oxyacetaldehyde, cyclamen aldehyde, hydroxycitronellal, lilial and bourgeonal; the ketones include, for example, the ionones, $\alpha$-isomethylionone and methyl cedryl ketone; the alcohols include, for example, anethol, citronellol, eugenol, isoeugenol, geraniol, linalool, phenyl ethyl alcohol and terpinol; and the hydrocarbons include mainly the terpenes and balsams. It is preferable, however, to use mixtures of various aromatic substances that together produce an attractive scent. Ethereal oils of relatively low volatility, which are chiefly used as aroma components, are also suitable as perfume oils, e.g. sage oil, camomile oil, clove oil, melissa oil, oil of cinnamon leaves, lime blossom oil, juniper berry oil, vetiver oil, olibanum oil, galbanum oil, labolanum oil and lavandin oil. Preference is given to the use of bergamot oil, dihydromyrcenol, lilial, lyral, citronellol, phenyl ethyl alcohol, $\alpha$-hexyl cinnamaldehyde, geraniol, benzyl acetone, cyclamen aldehyde, linalool, boisambrene forte, ambroxan, indole, hedione, sandelice, lemon oil, tangerine oil, orange oil, allyl amyl glycolate, cyclovertal, lavandin oil, muscatel sage oil, $\beta$-damascone, bourbon geranium oil, cyclohexyl salicylate, vertofix coeur, iso-E-Super, Fixolide NP, evernyl, iraldein gamma, phenylacetic acid, geranyl acetate, benzyl acetate, rose oxide, romillat, irotyl and floramat alone or in admixture with one another.

As colourants the substances that are suitable and permitted for cosmetic purposes, as compiled, for example, in the publication "Kosmetische Farbemittel" of the Farbstoffkommission der Deutschen Forschungsgemeinschaft, Verlag Chemie, Weinheim, 1984, pages 81 to 106 may be used. The colourants are usually used in concentrations of from 0.001 to 0.1 % by weight, based on the total mixture.

Typical examples of bacteria-inhibiting agents are preservatives that have a specific action against gram-positive bacteria, such as 2,4,4'-trichloro-2'-hydroxydiphenyl ether, chlorhexidine (1,6-di(4-chlorophenyl-biguanido)hexane) or TCC (3,4,4'-trichlorocarbanilide).

A large number of aromatic substances and ethereal oils also have antimicrobial properties. Typical examples are the active ingredients eugenol, menthol and thymol in clove oil, mint oil and thyme oil. A natural deodorizing agent of interest is the terpene alcohol farnesol (3,7,11-trimethyl-2,6,10-dodecatrien-1-ol), which is present in lime blossom oil. Glycerol monolaurate has also proved to be a bacteriostatic agent. The amount of the additional bacteria-inhibiting agents present is usually from 0.1 to 2 % by weight, based on the solids content of the cosmetic composition according to the present invention.

The cosmetic compositions according to the present invention may furthermore contain as adjuvants antifoams, such as silicones, structurants, such as maleic acid, solubilizers, such as ethylene glycol, propylene glycol, glycerol or diethylene glycol, opacifiers, such as latex, styrene/PVP or styrene/acrylamide copolymers, complexing agents, such as EDTA, NTA, $\beta$-alaninediacetic acid or phosphonic acids, propellants, such as propane/butane mixtures, $N_2O$, dimethyl ether, $CO_2$, $N_2$ or air, so-called coupler and developer components as oxidation dye precursors, thioglycolic acid and derivatives thereof, thiolactic acid, cysteamine, thiomalic acid or a-mercaptoethanesulfonic acid as reducing agents or hydrogen peroxide, potassium bromate or sodium bromate as oxidizing agents.

Insect repellents are for example, N,N-diethyl-m-toluamide, 1,2-pentanediol or insect repellent 3535.

Suitable self-tanning agents are dihydroxyacetone, erythrulose or mixtures of dihydroxyacetone and erythrulose.

The following examples are illustrative of the principles and practice of the present invention, although not limited thereto.

Examples

Materials and methods

Hydroxyphenyl-triazines

4,4'-[6-(4-methoxy-phenyl)-[1,3,5]-triazine-2,4-diyl]-bis-benzene-1,3-diol, CAS 1440-00-2
4-[4,6-di-(2,4-di-hydroxyphenyl)-1,3,5-triazine-2-yl]-benzene-1,3-diol, CAS 2125-23-7

Oxiranes

Oxirane mixture A: Oxirane, 2-[(C12-14-alkyloxy)methyl] derivs., CAS Registry Number 68609-97-2 (e.g. from Sigma-Aldrich)
Oxirane mixture B: Oxirane, 2-[(C11-15-alkyloxy)methyl] deriatives, technical grade, isomix C11-C15, mainly C13

Solubility testing

The UV absorber mixture is suspended in 1200mg solvent in a glass container (sonication bath, vortex shaker). A magnetic stirring bar is added. The container is closed and stirred over night at room temperature (20-35°C). It is always controlled that stirrer does not stick to the glass container.

Specification: clear solution

| | |
|---|---|
| Cosmetic solvents (INCI names): | C12-15 Alkyl Benzoate |
| | C12-15 Alkyl Benzoate |
| | Dibutyl Adipate |
| | Dicaprylyl Carbonate |

Determination of E (1%/1cm) at 343nm by UV spectroscopy:

Spectrophotometer Lamda 950S (or equivalent)
Cell Type: Quarz, 10 mm
Reference: 1.4-dioxane
Temperature: ca.25°C
Solvent: 1.4-dioxane, spectrophotometric grade

Preparation of the test solutions: About 25 mg of sample is weighed with a precision balance into a 100.0 ml (Vs) volumetric flask. It is filled up to the mark with 1.4-dioxane. 10.0 ml (V) of this solution is diluted to 100.0 ml (Vf) with 1.4-dioxane. The absorbance of this solution is measured between 290 and 450 nm.

Calculation of E (1%/1cm):

Weighing w = in mg

Total volume of stock solution Vs

Used volume of stock solution V

Final volume of solution Vf

Cell d = 10 mm

Wavelength maximum λ = 343nm

Measured absorbance at 343 nm A

$$E\ (1\%,1cm) = A_m \cdot \frac{Vs \cdot Vf * 10}{w \cdot V}$$

Molecular weight distribution by GPC

Method: Gel Permeation Chromatography with RI-Detection
Standards: EasiVial GPC/SEC Calibration Standards PSS Part.No: PL2010-0201 Agilent
Solvents: Tetrahydrofurane HPLC quality, diethanolamine puriss p.a.
Apparatus: Malvern Viscotek with RI-Detector
Chromatography conditions: Column1: PSS SDV 100 000 A, 8X300mm, 5u
Column2: PSS SDV 1000 A, 8x300mm, 5u
Oven temperature: 40°C
Mobile Phase: Tetrahydrofurane + 3.7g/L DEA
Flow: 1.0 ml/min
Sample concentration: approx. 2mg/ml in the same solvent mixture as the mobile phase.
Calibration: Conventional calibration homopolymeres. Polystyrene reference samples.

Example A1: Preparation of UV absorber mixture UV-TRIOX-01 (1.1)

Tentative structure

| | |
|---|---|
| UV-TRIOX-01 | 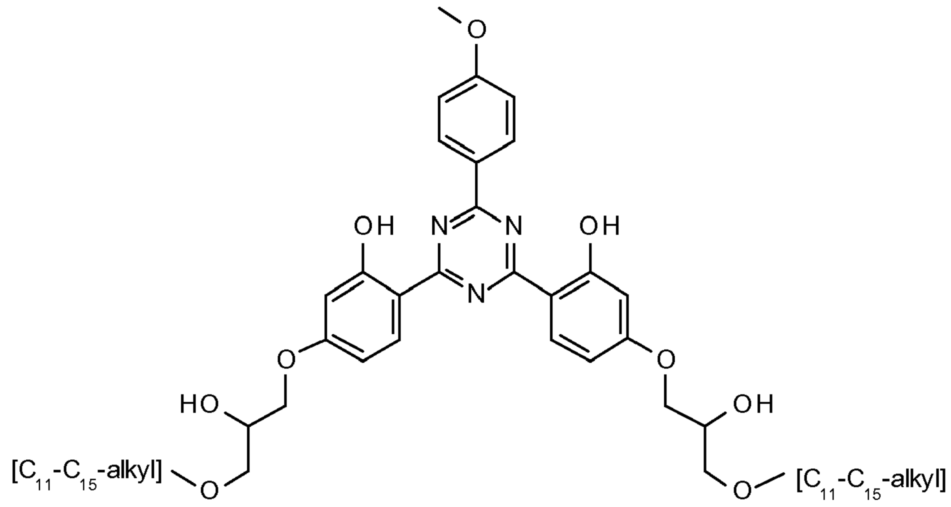 |
| | 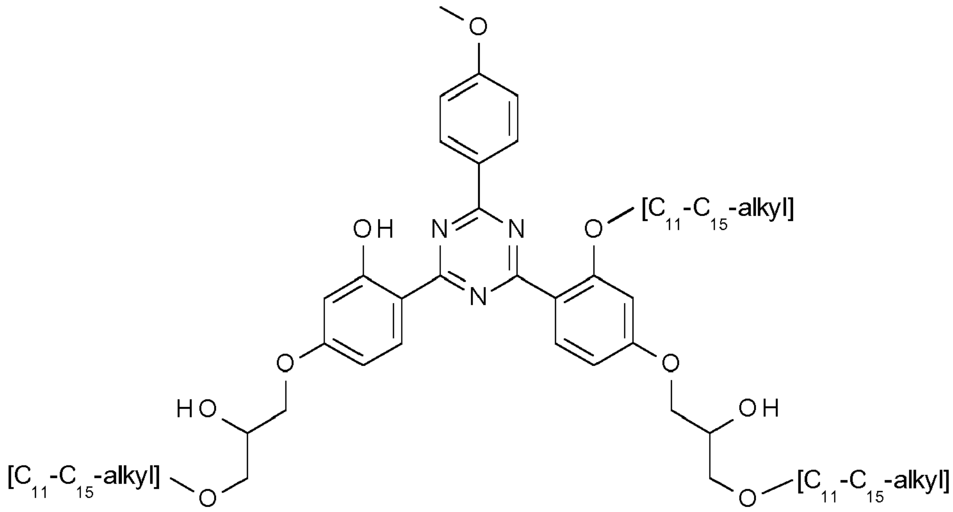 |

4,4'-[6-(4-methoxy-phenyl)-[1,3,5]-triazine-2,4-diyl]-bis-benzene-1,3-diol (60.04g, 149 mmol) is suspended in N,N-dimethylformamide (265g). Sodium methoxide solution (3.0 g, 30% in MeOH, 17mmol) is added and the mixture is heated to 120°C. To the resulting brown suspension oxirane mixture B, (99.5g, approx. 330mmol) is added dropwise over a two-hour period. The stirring is continued at 120°C for 20 hours. The reaction mixture is cooled to 25°, filtered over a bed of diatomaceous earth, and evaporated in vacuo. The crude material (162 g) is dissolved in 649g of toluene. 16.1g of acetic acid is added und the solution stirred vigorously for 15 minutes. Water (325g) is added and the stirring is continued for 30 minutes. The organic phase is filtered over a bed of diatomaceous earth and evaporated in vacuo. The ultraviolet radiation absorbing composition (1.1) is obtained as sticky brown oil (137.3g)

| UV (dioxane) | | GPC | | Solubility | |
|---|---|---|---|---|---|
| Wavelength [nm] | E [1%, 1cm] | Peak RV - (ml) | 18.547 | Solvent | % by weight |
| 300 | 380 | Mw - (Daltons) | 1'112 | C12-15 alkyl benzoate | >40 |
| 306 | 398 | Mw / Mn | 1.347 | Dibutyl adipate | >40 |
| 320 | 413 | | | Dicaprylyl carbonate | >40 |
| 328 | 408 | | | | |
| 340 | 400 | | | | |
| 344 | 385 | | | | |

(continued)

| UV (dioxane) | | GPC | | Solubility | |
|---|---|---|---|---|---|
| Wavelength [nm] | E [1%, 1cm] | Peak RV - (ml) | 18.547 | Solvent | % by weight |
| 360 | 267 | | | | |
| 380 | 43 | | | | |
| 400 | 4 | | | | |

Example A2 Preparation of Ultraviolet radiation absorbing composition UV-TRIOX-02 (2.1)

Tentative structure

| | |
|---|---|
| UV-TRIOX-02 | 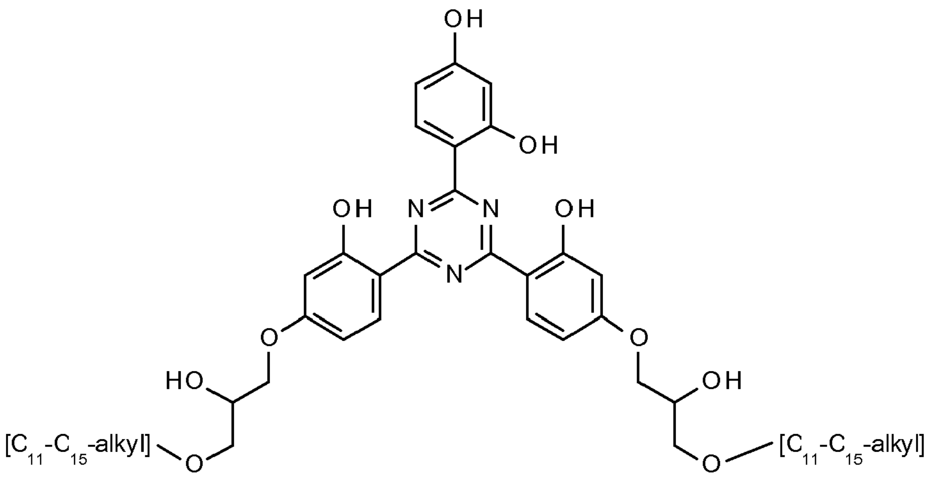 |
| | 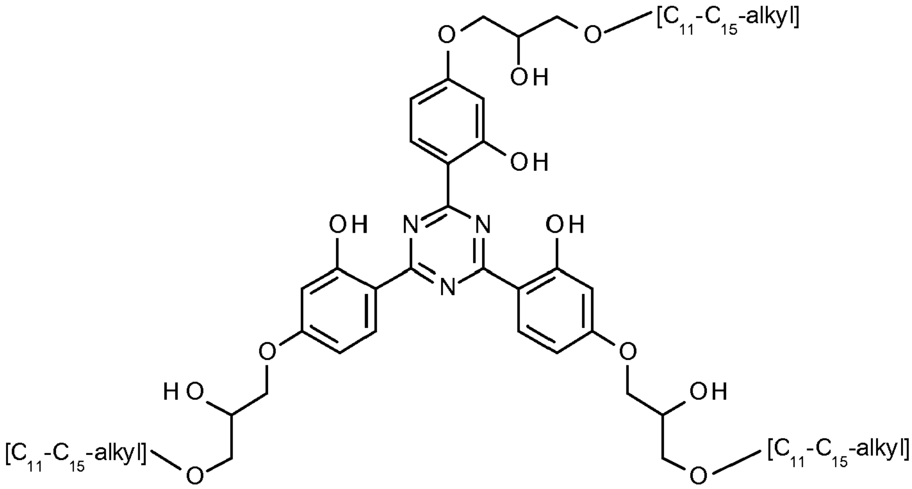 |

(continued)

| | |
|---|---|
| | 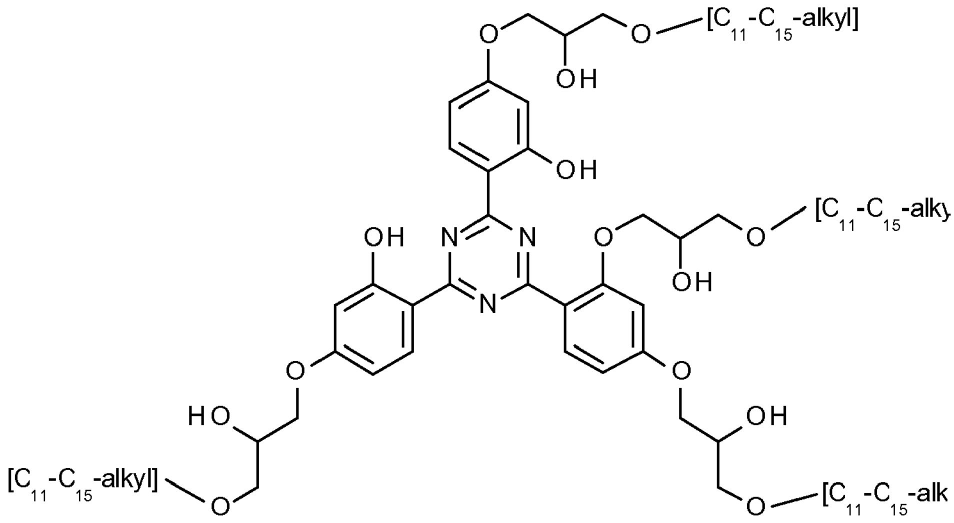 |

4-[4,6-di-(2,4-di-hydroxyphenyl)-1,3,5-triazin2-yl]-benzene-1,3-diol (71.1 g, 174mmol) is suspended in N,N-dimethylformamide (220g). Sodium methoxide solution (6.47g, 30% in MeOH, 36mmol) is added. The resulting yellow suspension is heated to 115°. At 115-120°, oxirane mixture B (158.6g, approx. 526 mmol) is added dropwise over a two-hour period. Temperature is increased to 140° and stirring is continued for 18 hours. The reaction mixture is cooled to 25°, turbidities were filtered of and the filtrate is evaporated in vacuo. The crude material (238g) is obtained as a dark brown oil. The crude product is dissolved in 714g of heptane. 23.9g of acetic acid is added. The heptane solution is extracted twice with methanol (~240g per extraction). The phases are separated and the heptane solution is evaporated in vacuo. The ultraviolet radiation absorbing composition (2.1) is obtained as sticky brown oil (186.5g).

| UV (dioxane) | | GPC | | Solubility | |
|---|---|---|---|---|---|
| Wavelength [nm] | E [1%, 1cm] | Peak RV - (ml) | 18.077 | Solvent | % by weight |
| 300 | 283 | Mw - (Daltons) | 1’653 | C12-15 alkyl benzoate | >40 |
| 306 | 296 | Mw / Mn | 2.215 | Dibutyl adipate | >40 |
| 320 | 271 | | | Dicaprylyl carbonate | >40 |
| 328 | 408 | | | | |
| 340 | 444 | | | | |
| 344 | 486 | | | | |
| 360 | 463 | | | | |
| 380 | 148 | | | | |
| 400 | 12 | | | | |

UV Spectra

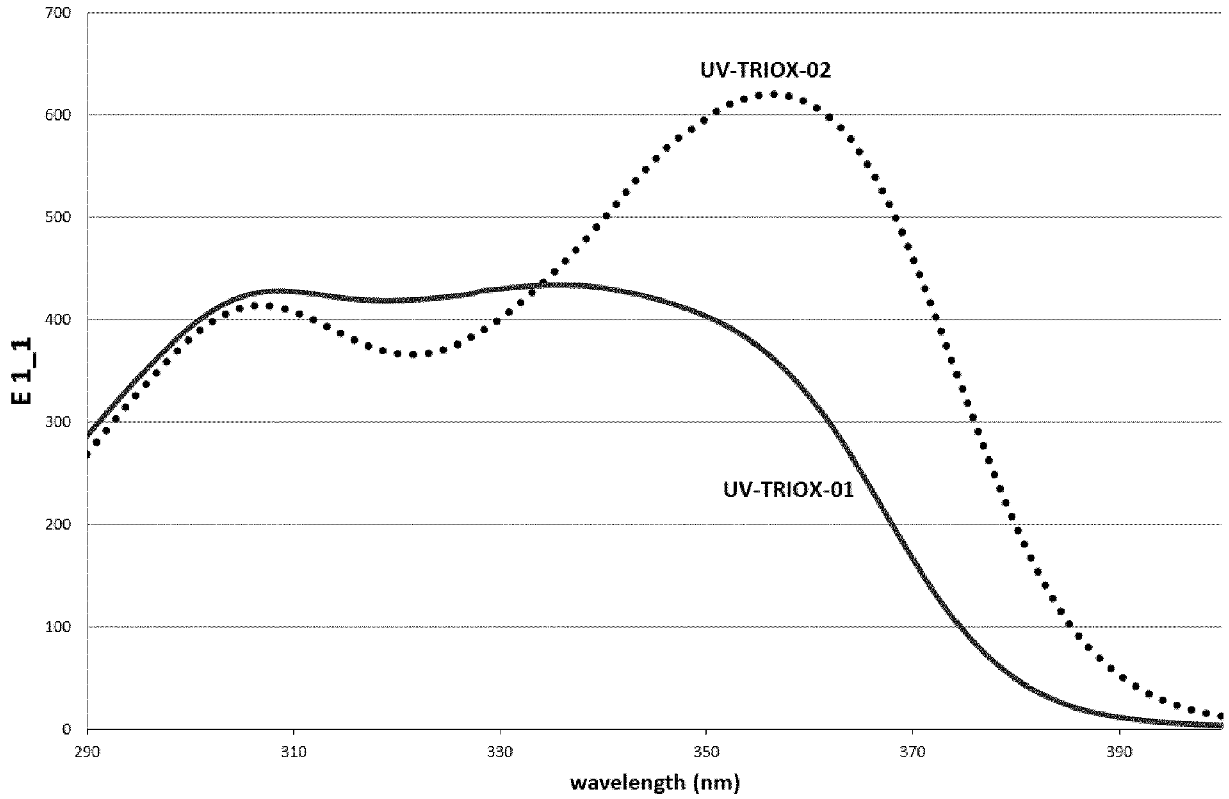


The solubility of Tinosorb S was determined as described in SOFW-Journal, 139, 7-2013, page 7-14.

Excellent solubility in typical Sun Care emollients, soluble in both low polar and high polar oils

| | UV-TRIOX-01 | UV-TRIOX-02 | Tinosorb S (Bis-Ethylhexyloxyphenol Methoxyphenyl Triazine) |
|---|---|---|---|
| Emollient | Solubility [% w/w 25°C] | Solubility [% w/w 25°C] | Solubility [% w/w 25°C] |
| Finsolv EB: Ethylhexyl Benzoate | >30% | >30% | 17% |
| Cetiol AB (C12-15 Alkyl Benzoate) | >30% | >30% | 10% |
| Cetiol B (Dibutyl Adipate) | >30% | >30% | 12% |
| Myritol 318 (Caprylic/Capric Triglyceride) | >30% | >25%<30% | 5% |
| Cetiol CC (Dicaprylyl Carbonate) | >30% | >30% | 9% |
| Dub DBS (Dibutyl Sebacate) | >30% | >30% | 9% |
| Cetiol Ultimate (Undecane and Tridecane) | >30% | >30% | 1% |
| Myritol 331 (Cocoglycerides) | >30% | >30% | 5% |

Photostability

For the determination of photodegradation the so-called "plate-test" is employed: 2 $\mu$l/cm$^2$ of the formulations are spread on quartz plates (sand-blasted surface of 2.8 cm$^2$), and rinsed off the plates after 0, 5, 10, 20, and 50 MED of UV irradiation using an Atlas CPS+ solar simulator.

Highly photostable ultraviolet radiation absorbing composition, no degradation detectable after 50 MED irradiation of a 2 mg/cm2 film containing 2% UV filter

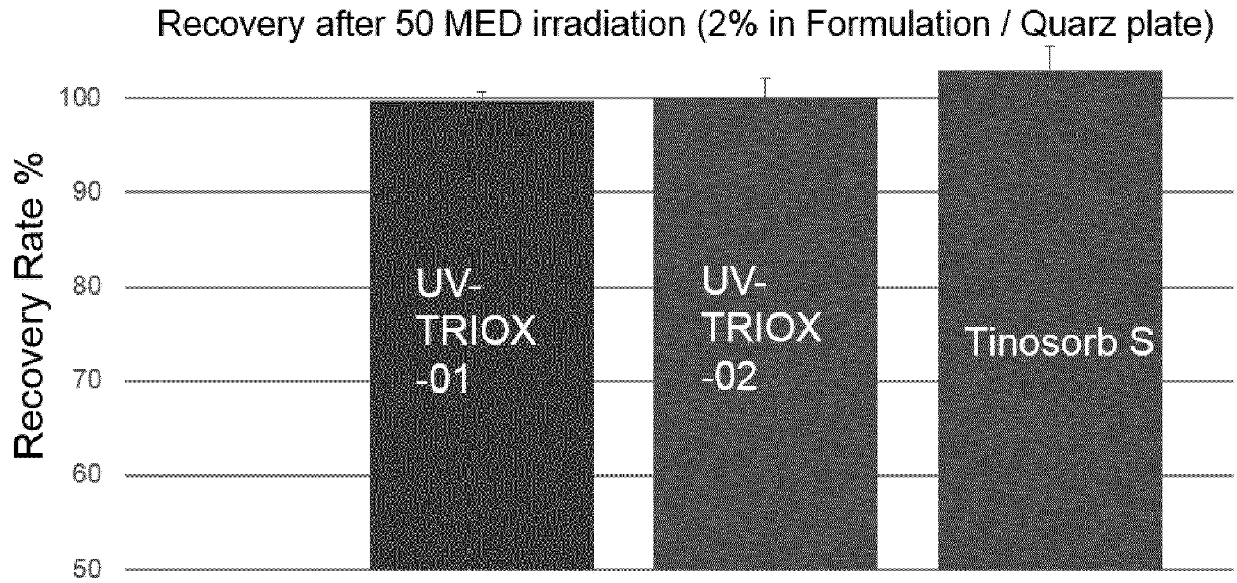


Formulation examples

The SPF and UVA-PF are calculated using the BASF Sunscreen Simulator software.

Calculation of the SPF

The sunburn protection factor (SPF) simulation makes use of the formalism first introduced by Sayre in 1979 [1], by which an average of the inverse transmission (1/T) of the respective sunscreen in the spectral range between 290 and 400 nm is calculated, including weighting with the irradiance spectrum of a UV source, Ss($\lambda$), and the erythemal action spectrum, Ser($\lambda$):

$$SPF_{invitro} = \frac{\sum_{290}^{400} s_{er}(\lambda)\cdot S_s(\lambda)}{\sum_{290}^{400} s_{er}(\lambda)\cdot S_s(\lambda)\cdot T(\lambda)}$$

The BASF Sunscreen Simulator calculates two values for the SPF referring to different irradiance spectra of the UV source. The first value is calculated for clinical conditions as defined in the international test method [2], using a source where the visible light is filtered out, which leads to an irradiance spectrum with a much lower fraction of UVA compared to natural sunlight. The second SPF value is calculated for outdoor conditions using a solar light spectrum occurring midday in midsummer at 40°N [3]. The erythemal action spectrum in both cases was taken from ref. [4]. Whereas irradiance data Ss($\lambda$) and erythemal action spectrum Ser($\lambda$) data are taken from literature, the transmittance T($\lambda$) has to be calculated for each composition of UV filters. In order to generate relevant transmission data, mixed absorbance spectra are calculated according to the amounts and UV spectroscopic performances of the filters, based on an average optical pathlength of 20 $\mu$m (corresponding approximately to an application amount of 2 mg/cm$^2$). Further, the irregularity of the sunscreen film is taken into consideration. A step film model for this purpose had been described by O'Neill in 1984 [5], which had been employed in the very first version of the sunscreen simulator (2002). In the previous version of this software (2006), an exponential distribution model with two parameters had been chosen [6]. This present

version makes use of the continuous height distribution model based on the gamma distribution [7]. As in the former versions, the parameter of this model was calibrated using standard sunscreen formulations with well-known SPF data. In addition, the calculation takes photoinstabilities of the single filters into account [8] as well as stabilizing and destabilizing effects from inter-molecular interactions [9]. Also the effect of the distribution of the filters in the oil and the water phase of the formulation is taken into consideration for the simulation procedure [10]. Besides the exact calculated value, the program indicates a value rounded in accordance with the international SPF test method [11].

Calculation of the UVA-PF

The in vitro method employed for determination of the UVA-PF has been developed by COLIPA [15]. In order to simulate this method, the film irregularity model was not used, but a relationship between parent film extinction and extinction of the film on the substrate [16]. Thus, an in vitro extinction spectrum is simulated, resulting in a simulated in vitro SPF. This may differ from the simulated in vivo SPF. The program then performs an adjustment of the corresponding simulated in vitro transmission spectrum, such that the simulated in vitro SPF matches the simulated in vivo SPF by adjusting the parameter C, which is the also used in the following equation for calculating the UVA-PF

$$UVA-PF=\frac{\sum_{320}^{400} s_{PPD}(\lambda)\cdot S_{UVA}(\lambda)}{\sum_{320}^{400} s_{PPD}(\lambda)\cdot S_{UVA}(\lambda)\cdot T(\lambda)^{C}} \qquad \text{(in vitro)}$$

The irradiation step involved in this method [15] is then simulated as well, leading to the indicated result of the UVA-PF (in vitro). The program then calculates the ratio of UVA-PF/SPF (EC-ratio) for both, the in vivo and the in vitro result of the UVA-PF (but only the in ratio for the in vitro case is shown). If one of these results fulfills the requirements of the EC recommendation [17], the UVA-logo shows up at the right hand side of the results sheet. Additionally, a label SPF value may be put in by the user, to which the corresponding adjustment of the parameter C is then performed and changes due to the irradiation step are considered as well. Again, the UVA-PF/SPF ratio is calculated, now using of course the label SPF what has been put in by the user, and the correspondingly obtained value of the in UVA-PF. If the ratio fulfills the EC requirement, the UVA logo appears at the right hand side. An additional requirement for the UVA logo is that the critical wavelength must be equal or higher than 370 nm.

References

1. R. M. Sayre, P. P. Agin, G. J. LeVee, E. Marlowe. A comparison of in vivo and in vitro testing of sunscreening formulas, Photochem. Photobiol. 29 (1979) 559 - 566
2. COLIPA: International Sun Protection Factor (SPF) Test Method. European Cosmetic Toiletries and Perfumery Association - COLIPA, Brussels (2003)
3. B. L. Diffey, J. Robson. A new substrate to measure sunscreen protection factors throughout the ultraviolet spectrum, J. Soc. Cosmet. Chem. 40 (1989) 127 - 133
4. A. F. McKinlay, B. L. Diffey: A reference action spectrum for ultraviolet-induced erythema inhuman skin. CIE Journal 6 (1987), 17 - 22
5. J. J. O'Neill, Effect of film irregularities on sunscreen efficacy, J.Pharm. Sci. 73, 888 - 891 (1984)
6. B. Herzog, Prediction of Sun Protection Factors and UV-A Parameters by Calculation of UV Transmissions Through Sunscreen Films of Inhomogenous Surface Structure, Chapter 44 in: Sunscreens: Regulations and Commercial Development (3rd edition), Edited by N. Shaath. Cosmetic Science and Technology Series, Vol 28; Taylor & Francis Group, Boca Raton, FL, 2005,p.881-900
7. L. Ferrero, M. Pissavini, S. Marguerie, L. Zastrow: Efficiency of a continuous height distribution model of sunscreen film geometry to predict a realistic sun protection factor. J. Cosmet. Sci. 54(2003) 463 - 481
8. M. Wloka, R. F. M. Lange, H. Flösser-Müller: An in vitro SPF Screening Approach Considering the Photostability of the UV Filters. Proc. Int. Sun Protection Conference, London 2005
9. B. Herzog, S. Mongiat, K. Quass, C. Deshayes, "Prediction of Sun Protection Factors and UVA Parameters by Using a Calibrated Step Film Model", J. Pharm. Sc. 93, 1780 - 1795 (2004)
10. B. Herzog et al., publication in preparation
11. M. Brown, SPF Testing in Europe, The International SPF Test Method, Chapter 39 in: Sunscreens: Regulations

and Commercial Development (3rd edition), Edited by N. Shaath. Cosmetic Science and Technology Series, Vol 28; Taylor & Francis Group, Boca Raton, FL, 2005,p.779-806
15. COLIPA method for the in vitro determination of UVA protection provided by sunscreen products.2007, The European Cosmetics Toiletry and Perfumery Association - COLIPA, Rue de la Loi223/2, B-1040 Bruxelles
16. Bernd Herzog, Models for the Calculation of Sun Protection Factors and Parameters Characterizing the UVA Protection Ability of Cosmetic Sunscreens, in: Colloids in Cosmetics and Personal Care, ed. Tharwart F. Tadros, Vol. 4., Wiley-VCH, Weinheim 2008
17. European Commission Recommendation on the efficacy of sunscreen products and the claims made relating thereto, OJ L265, 20067647/EC, 39 - 43

| Example B1: Sun Care Line Cream | | | | | |
|---|---|---|---|---|---|
| Phase | Ingredients | INCI | Weight % | | Function |
| | | | a | b | |
| A | Emulgade® 165 | Glyceryl Stearate, PEG-100 Stearate | 2.00 | 2.00 | Emulsifier (O/W) |
| | Cetiol® B | Dibutyl Adipate | 3.00 | 3.00 | Emollient |
| | Finsolv TPP | C12-15 Alkyl Benzoate,) Dipropylene Glycol Dibenzoate, PPG-15 Stearyl Ether Benzoate | 3.00 | 3.00 | Emollient |
| | Lanette® 18 | Stearyl Alcohol | 1.00 | 1.00 | Consistency agent |
| | Lanette® O | Cetearyl Alcohol | 1.00 | 1.00 | Consistency agent |
| | Uvinul® N 539T | Octocrylene | 10.00 | 10.00 | UV-B filter |
| | Neo Heliopan OS | Ethylhexyl Salicylate | 5.00 | 5.00 | UV-B filter |
| | Eusolex 9020 | Butyl Methoxydibenzoylmethane | 4.00 | 4.00 | UV-A filter |
| | UV-TRIOX-01 | | 2.00 | | |
| | UV-TRIOX-02 | | | 2.00 | |
| B | MT-100 Z | Titanium Dioxide (nano), Aluminum Hydroxide, Stearic Acid | 2.70 | 2.70 | UV-B filter |
| | Water, demin. | Aqua | 46.75 | 46.75 | |
| | Amphisol K | Potassium Cetyl Phosphate | 2.80 | 2.80 | Emulsifier (O/W) |
| | Tinocare® GL | Sclerotium Gum | 1.00 | 1.00 | Humectant |
| | Keltrol CG-RD | Xanthan Gum | 0.25 | 0.25 | Rheology modifier |
| C | Edeta® BD | Disodium EDTA | 0.20 | 0.20 | Complexing agent |
| D | Xiameter PMX-0246 Cyclohexasiloxane | Cyclohexasiloxane, Cyclopentasiloxane | 2.00 | 2.00 | |
| E | Tinovis® ADE | Sodium Acrylates Copolymer, Hydrogenated Polydecene, PPG-1 Trideceth-6 | 0.50 | 0.50 | Rheology modifier |

(continued)

| Example B1: Sun Care Line Cream | | | | | |
|---|---|---|---|---|---|
| Phase | Ingredients | INCI | Weight % | | Function |
| | | | a | b | |
| F | Tinosorb® M | Methylene Bis-Benzotriazolyl Tetramethylbutylphenol (nano), Aqua, Decyl Glucoside, Propylene Glycol, Xanthan Gum | 12.00 | 12.00 | Broad spectrum UV filter |
| | Preservative | | qs | qs | Preservative |
| G | Perfume | Parfum | qs | qs | Fragrance |
| | Sodium Hydroxide (10% solution) | Sodium Hydroxide | 0.80 | 0.80 | pH Adjustment |
| Performance (BASF sunscreen simulator) | | | | a | b |
| SPF in silico | | | | 51.9 | 51.1 |
| UVA-PF in silico | | | | 29.5 | 31.6 |
| Manufacturing Process | | | | | |
| Heat up phase A to 75 -80°C without Titanium Dioxide.<br>At 75°C, add Titanium Dioxide, homogenize until homogeneously dispersed.<br>Heat up phase B without Keltrol RD and Amphisol K.<br>At 75°C add Keltrol RD and Amphisol K to phase B and mix until homogeneous.<br>Incorporate phase A into phase B under homogenization (Ultra Turrax type device). Mix until homogeneous.<br>Cool down under stirring. At 60°C, add phase C, mix until homogeneous then add phase D and homogenize. Cool down to room temperature, then add phase E and mix until homogeneous. Add phase F, finally adjust pH with phase G. | | | | | |

| Example B2: Sun Care Line Cream | | | | | |
|---|---|---|---|---|---|
| Phase | Ingredients | INCI | Weight % | | Function |
| | | | a | b | |
| A | Cetiol® AB | C12-15 Alkyl Benzoate | 14.00 | | Emollient |
| | DUB DIS | Diisopropyl Sebacate | 8.00 | | Emollient |
| | Uvinul® T 150 | Ethylhexyl Triazone | 3.00 | | UV-B filter |
| | Eusolex 9020 | Butyl Methoxydibenzoylmethane | 3.00 | 3.00 | UV-A filter |
| | UV-TRIOX-01 | | 2.50 | | |
| | UV-TRIOX-02 | | | 2.50 | |

(continued)

| Example B2: Sun Care Line Cream | | | | | |
|---|---|---|---|---|---|
| Phase | Ingredients | INCI | Weight % | | Function |
| | | | a | b | |
| B | Water, demin. | Aqua | 37.45 | 37.45 | |
| | Butylene Glycol | Butylene Glycol | 6.00 | 6.00 | Humectant |
| | Tinocare® GL | Sclerotium Gum | 3.00 | 3.00 | Humectant |
| | Glycerin | Glycerin | 1.00 | 1.00 | Humectant |
| | Carbopol Ultrez 10 Polymer | Carbomer | 0.40 | 0.40 | Rheology modifier |
| | Pemulen TR-2 Polymeric Emulsifier | Acrylates/C10-30 Alkyl Acrylate Crosspolymer | 0.30 | 0.30 | Rheology modifier |
| | Edeta® BX Powder | Tetrasodium EDTA | 0.20 | 0.20 | Complexing agent |
| | Keltrol RD | Xanthan Gum | 0.15 | 0.15 | Stabilizer |
| C | AMP-Ultra PC 2000 | Aminomethyl Propanol | qs | qs | pH Adjustment |
| D | Eusolex 232 | Phenylbenzimidazole Sulfonic Acid | 2.00 | 2.00 | UV-B filter |
| | Water, demin. | Aqua | 5.00 | 5.00 | |
| | AMP-Ultra PC 2000 | Aminomethyl Propanol | qs | qs | pH Adjustment |
| E | Tinosorb® M | Methylene Bis-Benzotriazolyl Tetramethylbutylphenol (nano), Aqua, Decyl Glucoside, Propylene Glycol, Xanthan Gum | 14.00 | 14.00 | Broad spectrum UV filter |
| F | Perfume | Parfum | qs | qs | Fragrance |
| | Preservative | | qs | qs | Preservative |

| Performance (BASF sunscreen simulator) | a | b |
|---|---|---|
| SPF in silico | 47.3 | 46.3 |
| UVA-PF in silico | 17.6 | 18.9 |

| Manufacturing Process |
|---|
| Heat up phase A to 40°C, keep at 40°C for a while, until phase completely melted.<br>Prepare phase B: disperse the Carbopol and Pemulen into water, let swell.<br>Adc the rest of phase B under stirring.<br>Add phase A into phase B under stirrer, then homogenize shortly (Ultra Turrax).<br>Neutralize with phase C under stirrer.<br>Add phase D (previously mixed) under homogenization.<br>Add phase E and F under stirrer.<br>Check pH 7.0. |

| Example B3: Water Resistant Sun Matrix with Tinosorb S Aqua | | | | | |
|---|---|---|---|---|---|
| Phase | Ingredients | INCI | Weight % | | Function |
| | | | a | b | |
| A | Eumulgin® Prisma | Disodium Cetearyl Sulfosuccinate | 3.00 | 3.00 | Emulsifier (O/W) |
| | Cetiol® B | Dibutyl Adipate | 10.00 | 10.00 | Emollient |
| | DUB DIS | Diisopropyl Sebacate | 5.00 | 5.00 | Emollient |
| | Crodamol PMP | PPG-2 Myristyl Ether Propionate | 5.00 | 5.00 | Emollient |
| | Uvinul® A Plus | Diethylamino Hydroxybenzoyl Hexyl Benzoate | 8.00 | 8.00 | UV-A filter |
| | Uvinul® T 150 | Ethylhexyl Triazone | 3.00 | 3.00 | UV-B filter |
| | UV-TRIOX-01 | | 2.00 | | |
| | UV-TRIOX-02 | | | 2.00 | |
| B | Water, demin. | Aqua | 34.00 | 34.00 | |
| | Edeta® BD | Disodium EDTA | 0.20 | 0.20 | Complexing agent |
| | Amphisol K | Potassium Cetyl Phosphate | 0.50 | 0.50 | Emulsifier (O/W) |
| | Keltrol CG-T | Xanthan Gum | 0.50 | 0.50 | Stabilizer |
| C | Xiameter PMX-0246 Cyclohexasiloxane | Cyclohexasiloxane, Cyclopentasiloxane | 4.00 | 4.00 | Emollient |
| | KSG-16 | Dimethicone, Dimethicone/ Vinyl Dimethicone Crosspolymer | 2.00 | 2.00 | Skin feel modifier |
| D | Dry-Flo AF | Corn Starch Modified | 2.00 | 2.00 | Skin feel modifier |
| E | Water, demin. | Aqua | 6.00 | 6.00 | |
| | Eusolex 232 | Phenylbenzimidazole Sulfonic Acid | 3.00 | 3.00 | UV-B filter |
| | Tris Amino Ultra Pur | Tromethamine | 1.80 | 1.80 | |
| F | Tinosorb S Aqua | Aqua, Bis-Ethylhexyloxyphenol Methoxyphenyl Triazine, Polymethyl Methacrylate, Sodium Laureth Sulfate, Aminomethyl Propanol | 10.00 | 10.00 | Broad spectrum UV filter |
| | Preservative | | qs | qs | Preservative |
| | Perfume | Parfum | qs | qs | Fragrance |

| Performance (BASF sunscreen simulator) | a | b |
|---|---|---|
| SPF in silico | 47.7 | 49.2 |
| UVA-PF in silico | 24.9 | 27.5 |

| Manufacturing Process |
|---|
| Heat up phase A to 75°C under stirring. |

(continued)

| Manufacturing Process |
|---|
| Heat up phase B without Amphisol K and Keltrol CG-T to 75°C under stirring. Then add Keltrol CG-T into phase B under homogenizer.<br>Afterwards add Amphisol K, homogenize.<br>Add phase A into B under homogenizer.<br>Then add phase C previously mixed.<br>Add phase D, homogenize.<br>Cool down to room temperature under continuous stirring.<br>Add phase E previously mixed.<br>Finally add the ingredients of phase F and adjust final pH value to > 7. |

| Example B4: Clear UV Protect Spray | | | | | |
|---|---|---|---|---|---|
| Phase | Ingredients | INCI | Weight % | | Function |
| | | | a | b | |
| A | Cetiol® AB | C12-15 Alkyl Benzoate | 23.00 | 23.00 | Emollient |
| | Cetiol® CC | Dicaprylyl Carbonate | 5.00 | 5.00 | Emollient |
| | Neo Heliopan E 1000 | Isoamyl p-Methoxycinnamate | 10.00 | 10.00 | UV-B filter |
| | Uvinul® A Plus | Diethylamino Hydroxybenzoyl Hexyl Benzoate | 10.00 | 10.00 | UV-A filter |
| | UV-TRIOX-01 | | 3.50 | | |
| | UV-TRIOX-02 | | | 3.50 | |
| | Uvinul® T 150 | Ethylhexyl Triazone | 2.00 | 2.00 | UV-B filter |
| | Tinogard® TT | Tetradibutyl Pentaerithrityl Hydroxyhydrocinnamate | 0.03 | 0.03 | Antioxidant |
| B | Xiameter PMX-0345 Cyclosiloxane Blend | Cyclopentasiloxane, Cyclohexasiloxane | 10.00 | 10.00 | |
| C | Vitamin E-Acetate Care | Tocopheryl Acetate | 0.20 | 0.20 | Active ingredient |
| D | Dermacryl-79 | Acrylates/Octylacrylamide Copolymer | 0.50 | 0.50 | Film forming agent |
| | Ethanol | Alcohol | 35.77 | 35.77 | Solvent |

| Performance (BASF sunscreen simulator) | a | b |
|---|---|---|
| SPF in silico | 47.7 | 46.8 |
| UVA-PF in silico | 19.8 | 22.2 |

| Manufacturing Process |
|---|
| Heat phase A to 80°C, let stir for a while. Cool down to room temperature.<br>Add phase B then phase C to phase A.<br>Mix part D. Incorporate phase D into A/B/C |

| Example B5: Water-Like Sun Gel PA++ | | | | | |
|---|---|---|---|---|---|
| Phase | Ingredients | INCI | Weight % | | Function |
| | | | a | b | |
| A | Uvinul® MC 80 | Ethylhexyl Methoxycinnamate | 5.00 | 6.00 | UV-B filter |
| | Neo Heliopan OS | Ethylhexyl Salicylate | 3.00 | 5.00 | UV-B filter |
| | Uvinul® A Plus | Diethylamino Hydroxybenzoyl Hexyl Benzoate | 10.00 | 10.00 | UV-A filter |
| | UV-TRIOX-01 | | 10.00 | | |
| | UV-TRIOX-02 | | | 10.00 | |
| B | Water, demin. | Aqua | qs. 100 | qs. 100 | |
| | Butylene Glycol | Butylene Glycol | 2.50 | 2.50 | Humectant |
| | Mais PO4 PH "B" | Distarch Phosphate | 2.00 | 2.00 | Skin feel modifier |
| | Tinovis® GTC UP | Acrylates/Beheneth-25 Methacrylate Copolymer | 1.50 | 1.50 | Rheology modifier |
| C | Sodium Hydroxide (30% solution) | Sodium Hydroxide | qs | qs | pH Adjustment |
| D | Ethanol | Alcohol | 5.00 | 5.00 | Cooling agent |
| | Perfume | Parfum | qs | qs | Fragrance |
| | Preservative | | qs | qs | Preservative |

| Performance (BASF sunscreen simulator) | a | b |
|---|---|---|
| SPF in silico | 32.0 | 30.7 |
| UVA-PF in silico | 11.3 | 12.8 |

| Manufacturing Process |
|---|
| Heat phase A to 80°C.<br>Heat phase B to 80°C, stir 10 min at 80°C.<br>Add phase A into phase B under stirring. Homogenize shortly.<br>Add phase C under stirring.<br>Cool down to room temperature then add the ingredients of phase D in the listed order. |

| Example B6: Light Feeling Sun Care Lotion PA++ | | | | | |
|---|---|---|---|---|---|
| Phase | Ingredients | INCI | Weight % | | Function |
| | | | a | b | |
| A | Cetiol® CC | Dicaprylyl Carbonate | 5.00 | 5.00 | Emollient |
| | Cetiol® B | Dibutyl Adipate | 4.00 | 4.00 | Emollient |
| | Sensiva SC 50 | Ethylhexylglycerin | 0.50 | 0.50 | Auxiliary |
| | Uvinul® MC 80 | Ethylhexyl Methoxycinnamate | 10.00 | 10.00 | UV-B filter |
| | Uvinul® A Plus | Diethylamino Hydroxybenzoyl Hexyl Benzoate | 4.00 | 4.00 | UV-A filter |
| | UV-TRIOX-01 | | 2.00 | | |
| | UV-TRIOX-02 | | | 2.00 | |
| B | Water, demin. | Aqua | qs. 100 | qs. 100 | |
| | Glycerin | Glycerin | 3.00 | 3.00 | Humectant |
| | Tinovis® GTC UP | Acrylates/Beheneth-25 Methacrylate Copolymer | 2.00 | 2.00 | Rheology modifier |
| | Protectol® PE | Phenoxyethanol | 1.00 | 1.00 | Preservative |
| | Edeta® BD | Disodium EDTA | 0.20 | 0.20 | Complexing agent |
| | Keltrol CG-RD | Xanthan Gum | 0.20 | 0.20 | Rheology modifier |
| C | Sodium Hydroxide (30% solution) | Sodium Hydroxide | 0.25 | 0.25 | pH Adjustment |
| D | Tinosorb® S Aqua | Aqua, Bis-Ethylhexyloxyphenol Methoxyphenyl Triazine, Polymethyl Methacrylate, Sodium Laureth Sulfate, Aminomethyl Propanol | 10.00 | 5.00 | Broad spectrum UV Filter |
| | Tinosorb® M | Methylene Bis-Benzotriazolyl Tetramethylbutylphenol (nano), Aqua, Decyl Glucoside, Propylene Glycol, Xanthan Gum | 4.00 | 2.00 | Broad spectrum UV filter |
| E | Xiameter PMX-0246 Cyclohexasiloxane | Cyclohexasiloxane, Cyclopentasiloxane | 3.00 | 3.00 | Emollient |
| | Orgasol Caresse | Polyamide-5 | 3.00 | 3.00 | |
| | Perfume | Parfum | qs | qs | Fragrance |

| Performance (BASF sunscreen simulator) | a | b |
|---|---|---|
| SPF in silico | 33.7 | 33.2 |
| UVA-PF in silico | 10.5 | 11.5 |
| Adjust ph value to (25°C) | 6.00 - 7.00 | |

| Manufacturing Process |
|---|
| Heat up phase A to 80°C, then cool down to room temperature.<br>Mix phase B. |

(continued)

| Manufacturing Process |
|---|
| Incorporate phase A into phase B under stirring, homogenize (with Ultra-Turrax type device).<br>Add phase C (under Ultra-Turrax device).<br>Finally add the ingredients of phase D and E, homogenize for a short time. |

| Example B7: Plus Point Soft Cream with Tinosorb M | | | | | |
|---|---|---|---|---|---|
| Phase | Ingredients | INCI | Weight % | | Function |
| | | | a | b | |
| A | Emulgade® Sucro | Sucrose Polystearate, Hydrogenated Polyisobutene | 4.50 | 4.50 | Emulsifier (O/W) |
| | Eumulgin® Prisma | Disodium Cetearyl Sulfosuccinate | 2.00 | 2.00 | Emulsifier (O/W) |
| | Lanette® O | Cetearyl Alcohol | 1.00 | 1.00 | Consistency agent |
| | Cetiol® AB | C12-15 Alkyl Benzoate | 10.00 | 10.00 | Emollient |
| | Cetiol® B | Dibutyl Adipate | 10.00 | 10.00 | Emollient |
| | DUB DIS | Diisopropyl Sebacate | 5.00 | 5.00 | Emollient |
| | Uvinul® A Plus | Diethylamino Hydroxybenzoyl Hexyl Benzoate | 3.50 | 2.00 | UV-A filter |
| | Uvinul® T 150 | Ethylhexyl Triazone | 3.00 | 3.00 | UV-B filter |
| | UV-TRIOX-01 | | 5.00 | | |
| | UV-TRIOX-02 | | | 7.00 | |
| B | Water, demin. | Aqua | qs. 100 | qs.100 | |
| | Glycerin | Glycerin | 3.00 | 3.00 | Humectant |
| | Keltrol CG-RD | Xanthan Gum | 0.50 | 0.50 | Rheology modifier |
| | Edeta® BD | Disodium EDTA | 0.20 | 0.20 | Complexing agent |
| C | Eusolex 232 | Phenylbenzimidazole Sulfonic Acid | 3.00 | 3.00 | UV-B filter |
| | Water, demin. | Aqua | 6.00 | 6.00 | |
| | Tris Amino Ultra Pur | Tromethamine | qs | qs | |

(continued)

| Example B7: Plus Point Soft Cream with Tinosorb M | | | | | |
|---|---|---|---|---|---|
| Phase | Ingredients | INCI | Weight % | | Function |
| | | | a | b | |
| D | Tinosorb® M | Methylene Bis-Benzotriazolyl Tetramethylbutylphenol (nano), Aqua, Decyl Glucoside, Propylene Glycol, Xanthan Gum | 7.00 | 7.00 | Broad spectrum UV filter |
| | Techpolymer MBP 8 | Polymethyl Methacrylate | 2.00 | 2.00 | |
| | Orgasol Caresse | Polyamide-5 | 1.00 | 1.00 | |
| | Perfume | Parfum | qs | qs | Fragrance |
| | Preservative | | qs | qs | Preservative |

| Performance (BASF sunscreen simulator) | a | b |
|---|---|---|
| SPF in silico | 51.3 | 50.9 |
| UVA-PF in silico | 17.5 | 20.1 |

| Adjust ph value to (25°C) | > 7.00 |
|---|---|
| Manufacturing Process | |
| Heat up phase A and B separately with stirring to 80°C.<br>Add phase B to phase A under stirring, homogenize.<br>Add phase C under stirring.<br>Cool down under stirring, at 40°C add phase D. Continue stirring to room temperature. | |

| Example B8: O/W emulsion | | | | | |
|---|---|---|---|---|---|
| Phase | Ingredients | INCI | Weight % | | Function |
| | | | a | b | |
| A | Cetiol® AB | C12-15 Alkyl Benzoate | 20.00 | 20.00 | Emollient |
| | Cetiol® B | Dibutyl Adipate | 10.00 | 10.00 | Emollient |
| | Lanette® O | Cetearyl Alcohol | 1.50 | 1.50 | Consistency agent |
| | Lanette® 18 | Stearyl Alcohol | 1.00 | 1.00 | Consistency agent |
| | Uvinul® A Plus | Diethylamino Hydroxybenzoyl Hexyl Benzoate | 5.00 | 2.00 | UV-A filter |
| | Uvinul® T 150 | Ethylhexyl Triazone | 2.20 | 1.00 | UV-B filter |
| | UV-TRIOX-01 | | 10.00 | | |
| | UV-TRIOX-02 | | | 10.00 | |

(continued)

| Example B8: O/W emulsion | | | | | |
|---|---|---|---|---|---|
| Phase | Ingredients | INCI | Weight % | | Function |
| | | | a | b | |
| B | Water, demin. | Aqua | qs. 100 | qs. 100 | |
| | Glycerin | Glycerin | 3.00 | 3.00 | Humectant |
| | Amphisol K | | 2.50 | 2.50 | Emulsifier (O/W) |
| | Edeta® BD | Disodium EDTA | 0.20 | 0.20 | Complexing agent |
| | Keltrol CG-RD | Xanthan Gum | 0.15 | 0.15 | Stabilizer |
| C | Water, demin. | Aqua | 6.00 | 6.00 | |
| | Eusolex 232 | Phenylbenzimidazole Sulfonic Acid | 1.80 | 1.50 | UV-B filter |
| | Tris Amino Ultra Pur | Tromethamine | qs | qs | |
| D | Tinosorb® A2B | Tris-Biphenyl Triazine (nano), Aqua, Decyl Glucoside, Butylene Glycol, Disodium Phosphate, Xanthan Gum | 4.00 | 4.00 | UV-B / A II filter |
| E | Tinocare® WRP | Polyacrylate-27 | 1.00 | 1.00 | |
| | Orgasol 2002 EXD NAT COS Typ S | Nylon-12 | 2.00 | 2.00 | |
| | Preservative | | qs | qs | Preservative |
| | Perfume | Parfum | qs | qs | Fragrance |
| | Sodium Hydroxide (30% solution) | Sodium Hydroxide | qs | qs | pH Adjustment |

| Performance (BASF sunscreen simulator) | a | b |
|---|---|---|
| SPF in silico | 58.1 | 31.2 |
| UVA-PF in silico | 19.7 | 14.5 |

| Manufacturing Process |
|---|
| Heat phase A to 75°C under stirring, add $TiO_2$ homogenize with Ultra Turrax<br>Heat phase B without Amphisol K to 75°C under stirring<br>Add Amphisol K into phase B continue stirring 10 min<br>Add A into B under stirring, homogenize. Under stirring, add phase C.<br>Continue stirring to cool down to room temperature then add phase D and E Adjust pH to 6.8 - 7.0. |

| Example B9: Balanced Protection Lotion EHMC & OCR free with Tinosorb A2B | | | | | |
|---|---|---|---|---|---|
| Phase | Ingredients | INCI | Weight % | | Function |
| A | Cetiol® AB | C12-15 Alkyl Benzoate | 15.00 | 15.00 | Emollient |
| | DUB DIS | Diisopropyl Sebacate | 14.00 | 14.00 | Emollient |
| | Lanette® O | Cetearyl Alcohol | 1.50 | 1.50 | Consistency agent |
| | Lanette® 18 | Stearyl Alcohol | 1.00 | 1.00 | Consistency agent |
| | Sensiva SC 50 | Ethylhexylglycerin | 0.50 | 0.50 | Humectant |
| | Parsol 1789 | Butyl Methoxydibenzoylmethane | 3.00 | 3.00 | UV-A filter |
| | UV-TRIOX-01 | | 2.80 | | |
| | UV-TRIOX-02 | | | 2.80 | |
| | Uvinul® T 150 | Ethylhexyl Triazone | 2.50 | 2.50 | UV-B filter |
| B | Water, demin. | Aqua | qs.100 | qs. 100 | |
| | Glycerin | Glycerin | 3.00 | 3.00 | Humectant |
| | Amphisol K | Potassium Cetyl Phosphate | 2.50 | 2.50 | Emulsifier (O/W) |
| | Keltrol CG-RD | Xanthan Gum | 0.25 | 0.25 | Stabilizer |
| | Dissolvine NA-2 | Disodium EDTA | 0.20 | 0.20 | |
| C | Tinosorb® M | Methylene Bis-Benzotriazolyl Tetramethylbutylphenol (nano), Aqua, Decyl Glucoside, Propylene Glycol, Xanthan Gum | 13.00 | 13.00 | Broad spectrum UV filter |
| | Tinosorb® A2B | Tris-Biphenyl Triazine (nano), Aqua, Decyl Glucoside, Butylene Glycol, Disodium Phosphate, Xanthan Gum | 6.00 | 6.00 | UV-B / A II filter |
| D | Orgasol 2002 EXD NAT COS Typ S | Nylon-12 | 2.00 | 2.00 | |
| | Protectol® PE | Phenoxyethanol | 1.00 | 1.00 | Preservative |
| | Sodium Hydroxide (30% solution) | Sodium Hydroxide | qs | qs | pH Adjustment |

| Performance (BASF sunscreen simulator) | a | b |
|---|---|---|
| SPF in silico | 53.8 | 52.5 |
| UVA-PF in silico | 20.3 | 22.1 |

| Manufacturing Process |
|---|
| Heat up phase A to 75°C under stirring. |

(continued)

| Manufacturing Process |
|---|
| Heat up phase B without Amphisol K to 75°C under stirring.<br>At 75°C add Amphisol K into phase B, continue stirring.<br>Add phase A into phase B under stirring, homogenize.<br>Cool down to 40°C under stirring.<br>Add the ingredients of phase C and D under stirring Cool down to room temperature under stirring. |

Example B10: Water Resistant Plus Points with Tinosorb A2B

| Phase | Ingredients | INCI | Weight % | | Function |
|---|---|---|---|---|---|
| | | | a | b | |
| A | Cetiol® B | Dibutyl Adipate | 17.00 | 17.00 | Emollient |
| | Cetiol® AB | C12-15 Alkyl Benzoate | 3.00 | 3.00 | Emollient |
| | Lanette® O | Cetearyl Alcohol | 2.50 | 2.50 | Consistency agent |
| | Uvinul® A Plus | Diethylamino Hydroxybenzoyl Hexyl Benzoate | 5.00 | 4.00 | UV-A filter |
| | Uvinul® T 150 | Ethylhexyl Triazone | 3.00 | 3.00 | UV-B filter |
| | UV-TRIOX-01 | | 1.00 | | |
| | UV-TRIOX-02 | | | 1.00 | |
| B | Water, demin. | Aqua | qs.100 | qs.100 | |
| | Glycerin | Glycerin | 3.00 | 3.00 | Humectant |
| | Amphisol K | Potassium Cetyl Phosphate | 2.50 | 2.50 | Emulsifier (O/W) |
| | Edeta® BD | Disodium EDTA | 0.20 | 0.20 | Complexing agent |
| | Keltrol CG-RD | Xanthan Gum | 0.15 | 0.15 | Rheology modifier |
| C | Tinosorb® A2B | Tris-Biphenyl Triazine (nano), Aqua, Decyl Glucoside, Butylene Glycol, Disodium Phosphate, Xanthan Gum | 4.00 | 4.00 | UV-B / A II filter |
| | Preservative | | qs | qs | Preservative |

| Performance (BASF sunscreen simulator) | a | b |
|---|---|---|
| SPF in silico | 32.8 | 12.1 |
| UVA-PF in silico | 13.8 | 30.9 |

| Manufacturing Process |
|---|
| Heat phase A to 75°C under stirring.<br>Heat phase B without Amphisol K to 75°C under stirring. Add Amphisol K into phase B continue stirring. Add phase A into B under stirring, homogenize.<br>Cool down under stirring, below 40°C add phase C. Continue stirring. |

| Example B11: Plus Points Lotion | | | | | |
|---|---|---|---|---|---|
| Phase | Ingredients | INCI | Weight % | | Function |
| | | | a | b | |
| A | Eumulgin® Prisma | Disodium Cetearyl Sulfosuccinate | 0.80 | 0.80 | Emulsifier (O/W) |
| | Finsolv EB | Ethylhexyl Benzoate | 20.00 | 20.00 | |
| | Cetiol® AB | C12-15 Alkyl Benzoate | 10.00 | 10.00 | Emollient |
| | Lanette® O | Cetearyl Alcohol | 3.00 | 3.00 | Consistency agent |
| | Uvinul® A Plus | Diethylamino Hydroxybenzoyl Hexyl Benzoate | 9.00 | 9.00 | UV-A filter |
| | MT-100 Z | Titanium Dioxide (nano), Aluminum Hydroxide, Stearic Acid | 4.70 | 4.70 | UV-B filter |
| | Uvinul® T 150 | Ethylhexyl Triazone | 3.50 | 3.50 | UV-B filter |
| | UV-TRIOX-01 | | 3.00 | | |
| | UV-TRIOX-02 | | | 3.00 | |
| B | Water, demin. | Aqua | qs.100 | qs.100 | |
| | Glycerin | Glycerin | 3.00 | 3.00 | Humectant |
| | Amphisol K | Potassium Cetyl Phosphate | 2.50 | 2.50 | Emulsifier (O/W) |
| | Keltrol RD | Xanthan Gum | 0.50 | 0.50 | Stabilizer |
| | Edeta® BD | Disodium EDTA | 0.20 | 0.20 | Complexing agent |
| C | Water, demin. | Aqua | 6.00 | 6.00 | |
| | Eusolex 232 | Phenylbenzimidazole Sulfonic Acid | 4.00 | 4.00 | UV-B filter |
| | Tris Amino Ultra Pur | Tromethamine | 1.80 | 1.80 | |
| D | Preservative | | qs | qs | Preservative |
| | Perfume | Parfum | qs | qs | Fragrance |

| Performance (BASF sunscreen simulator) | a | b |
|---|---|---|
| SPF in silico | 64.9 | 63.9 |
| UVA-PF in silico | 22.3 | 24.7 |

| Manufacturing Process |
|---|
| Heat up phase A to 75°C under stirring, add Titanium dioxide, homogenize (Ultra Turrax).<br>Heat up phase B without Amphisol K to 75°C under stirring. Then add Amphisol K into phase B continue stirring for 10 more minutes. Add phase A into B under stirring, homogenize.<br>Add phase C under stirring.<br>Cool down to room temperature under continuous stirring, add phase D. Adjust pH value to at least 7. |

| Example B12: Photostable UVA Shield | | | | | |
|---|---|---|---|---|---|
| Phase | Ingredients | INCI | Weight % | | Function |
| | | | a | b | |
| A | Eumulgin® VL 75 | Lauryl Glucoside, Polyglyceryl-2 Dipolyhydroxystearate, Glycerin | 4.00 | 4.00 | Emulsifier (O/W) |
| | Myritol® 312 | Caprylic/Capric Triglyceride | 12.00 | 12.00 | Emollient |
| | Cetiol® B | Dibutyl Adipate | 8.00 | 8.00 | Emollient |
| | Cetiol® AB | C12-15 Alkyl Benzoate | 8.00 | 8.00 | Emollient |
| | Lanette® O | Cetearyl Alcohol | 2.00 | 2.00 | Consistency agent |
| | Uvinul® MC 80 | Ethylhexyl Methoxycinnamate | 10.00 | 10.00 | UV-B filter |
| | Uvinul® A Plus | Diethylamino Hydroxybenzoyl Hexyl Benzoate | 10.00 | 10.00 | UV-A filter |
| | UV-TRIOX-01 | | 6.00 | | |
| | UV-TRIOX-02 | | | 6.00 | |
| | Uvinul® T 150 | Ethylhexyl Triazone | 2.50 | 2.50 | UV-B filter |
| B | Water, demin. | Aqua | qs.100 | qs.100 | |
| | Glycerin | Glycerin | 3.00 | 3.00 | Humectant |
| | Veegum Ultra | Magnesium Aluminum Silicate | 1.50 | 1.50 | Stabilizer |
| | Lanette® E | Sodium Cetearyl Sulfate | 1.00 | 1.00 | Emulsifier (O/W) |
| | Keltrol CG-RD | Xanthan Gum | 0.30 | 0.30 | Rheology modifier |
| | Edeta® BD | Disodium EDTA | 0.05 | 0.05 | Complexing agent |
| C | Preservative | | qs | qs | Preservative |
| | Perfume | Parfum | qs | qs | Fragrance |

| Performance (BASF sunscreen simulator) | a | b |
|---|---|---|
| SPF in silico | 51.6 | 49.8 |
| UVA-PF in silico | 18.3 | 21.7 |
| Manufacturing Process | | |
| Heat up phase A to 80°C.<br>Phase B: heat up water and Veegum Ultra to 80°C, homogenize then add the rest of phase B, keep at 80°C. Add phase A into B under stirring, homogenize.<br>Cool down under stirrer to room temperature. Finally add phase C under continuous stirring. | | |

| Example B13: Water Resistant Sun Matrix with Tinosorb S Aqua | | | | | |
|---|---|---|---|---|---|
| Phase | Ingredients | INCI | Weight % | | Function |
| | | | a | b | |
| A | Eumulgin® Prisma | Disodium Cetearyl Sulfosuccinate | 3.00 | 3.00 | Emulsifier (O/W) |
| | Cetiol® B | Dibutyl Adipate | 10.00 | 10.00 | Emollient |
| | DUB DIS | Diisopropyl Sebacate | 5.00 | 5.00 | Emollient |
| | Crodamol PMP | PPG-2 Myristyl Ether Propionate | 5.00 | 5.00 | Emollient |
| | Uvinul® A Plus | Diethylamino Hydroxybenzoyl Hexyl Benzoate | 4.00 | 8.00 | UV-A filter |
| | Uvinul® T 150 | Ethylhexyl Triazone | 3.00 | 3.00 | UV-B filter |
| | UV-TRIOX-01 | | 3.00 | | |
| | UV-TRIOX-02 | | | 2.00 | |
| B | Water, demin. | Aqua | qs. 100 | qs. 100 | |
| | Edeta® BD | Disodium EDTA | 0.20 | 0.20 | Complexing agent |
| | Amphisol K | Potassium Cetyl Phosphate | 0.50 | 0.50 | Emulsifier (O/W) |
| | Keltrol CG-T | Xanthan Gum | 0.50 | 0.50 | Stabilizer |
| C | Xiameter PMX-0246 Cyclohexasiloxane | Cyclohexasiloxane, Cyclopentasiloxane | 4.00 | 4.00 | Emollient |
| | KSG-16 | Dimethicone, Dimethicone/ Vinyl Dimethicone Crosspolymer | 2.00 | 2.00 | Skin feel modifier |
| D | Dry-Flo AF | Corn Starch Modified | 2.00 | 2.00 | Skin feel modifier |
| E | Water, demin. | Aqua | 6.00 | 6.00 | |
| | Eusolex 232 | Phenylbenzimidazole Sulfonic Acid | 2.00 | 3.00 | UV-B filter |
| | Tris Amino Ultra Pur | Tromethamine | 1.80 | 1.80 | |
| | Tinosorb® S Aqua | Aqua, Bis-Ethylhexyloxy-phenol Methoxyphenyl Triazine, Polymethyl Methacrylate, Sodium Laureth Sulfate, Aminomethyl Propanol | 10.00 | 10.00 | Broad spectrum UV filter |
| | Preservative | | qs | qs | Preservative |
| | Perfume | Parfum | qs | qs | Fragrance |

| Performance (BASF sunscreen simulator) | a | b |
|---|---|---|
| SPF in silico | 31.2 | 30.4 |
| UVA-PF in silico | 10.5 | 12.3 |

| Manufacturing Process |
|---|
| Heat up phase A to 75°C under stirring. |

(continued)

| Manufacturing Process |
|---|
| Heat up phase B without Amphisol K and Keltrol CG-T to 75°C under stirring. Then add Keltrol CG-T into phase B under homogenizer. Afterwards add Amphisol K, homogenize.<br>Add phase A into B under homogenizer.<br>Then add phase C previously mixed.<br>Add phase D, homogenize.<br>Cool down to room temperature under continuous stirring.<br>Add phase E previously mixed.<br>Finally add the ingredients of phase F and adjust final pH value to > 7. |

Example B14: Photostable UVA with Tinosorb M lotion

| Phase | Ingredients | INCI | Weight % | | Function |
|---|---|---|---|---|---|
| | | | a | b | |
| A | Dehymuls® PGPH | Polyglyceryl-2 Dipolyhydroxystearate | 4.00 | 4.00 | Emulsifier (W/O) |
| | Cetiol® B | Dibutyl Adipate | 10.00 | 10.00 | Emollient |
| | Cetiol® AB | C12-15 Alkyl Benzoate | 5.00 | 5.00 | Emollient |
| | Lanette® O | Cetearyl Alcohol | 2.50 | 2.50 | Consistency agent |
| | Uvinul® A Plus | Diethylamino Hydroxybenzoyl Hexyl Benzoate | 10.00 | 10.00 | UV-A filter |
| | Uvinul® MC 80 | Ethylhexyl Methoxycinnamate | 9.50 | 9.50 | UV-B filter |
| | Uvinul® T 150 | Ethylhexyl Triazone | 2.50 | 2.50 | UV-B filter |
| | UV-TRIOX-01 | | 2.00 | | |
| | UV-TRIOX-02 | | | 2.00 | |
| B | Water, demin. | Aqua | qs. 100 | qs. 100 | |
| | Glycerin | Glycerin | 3.00 | 3.00 | Humectant |
| | Plantapon® LGC SORB | Sodium Lauryl Glucose Carboxylate (and) Lauryl Glucoside | 1.50 | 1.50 | Emulsifier (O/W) |
| | Cosmedia® SP | Sodium Polyacrylate | 0.80 | 0.80 | Rheology modifier |
| | Edeta® BD | Disodium EDTA | 0.20 | 0.20 | Complexing agent |
| | Keltrol CG-RD | Xanthan Gum | 0.15 | 0.15 | Rheology modifier |

(continued)

| Example B14: Photostable UVA with Tinosorb M lotion | | | | | |
|---|---|---|---|---|---|
| Phase | Ingredients | INCI | Weight % | | Function |
| | | | a | b | |
| C | Tinosorb® M | Methylene Bis-Benzotriazolyl Tetramethylbutylphenol (nano), Aqua, Decyl Glucoside, Propylene Glycol, Xanthan Gum | 4.00 | 4.00 | Broad spectrum UV filter |
| | Perfume | Parfum | qs | qs | Fragrance |
| | Preservative | | qs | qs | Preservative |

| Performance (BASF sunscreen simulator) | a | b |
|---|---|---|
| SPF in silico | 51.3 | 50.6 |
| UVA-PF in silico | 20.8 | 22.1 |

| Manufacturing Process |
|---|
| Heat phase A to 75°C under stirring.<br>Heat phase B to 75°C under stirring.<br>Add phase A into B under stirring, homogenize.<br>Cool down under stirring below 40°C add phase C. Continue stirring. |

| Example B15: UV Expert Summer Passion Fluid | | | | | |
|---|---|---|---|---|---|
| Phase | Ingredients | INCI | Weight % | | Function |
| | | | a | b | |
| A | Eumulgin® SG | Sodium Stearoyl Glutamate | 2.00 | 2.00 | Emulsifier (O/W) |
| | Tegosoft XC | Phenoxyethyl Caprylate | 8.00 | 8.00 | Emollient |
| | Cetiol® B | Dibutyl Adipate | 5.00 | 5.00 | Emollient |
| | Finsolv EB | Ethylhexyl Benzoate | 5.00 | 5.00 | |
| | Lanette® 18 | Stearyl Alcohol | 1.00 | 1.00 | Consistency agent |
| | Uvinul® A Plus | Diethylamino Hydroxybenzoyl Hexyl Benzoate | 7.00 | 7.00 | UV-A filter |
| | Uvinul® T 150 | Ethylhexyl Triazone | 3.00 | 3.00 | UV-B filter |
| | UV-TRIOX-01 | | 11.00 | | |
| | UV-TRIOX-02 | | | 11.00 | |
| B | Water, demin. | Aqua | qs. 100 | qs. 100 | |
| | Edeta® BD | Disodium EDTA | 0.20 | 0.20 | Complexing agent |
| | Rheocare® XG (Europe only) | Xanthan Gum | 0.50 | 0.50 | Rheology modifier |

(continued)

| Example B15: UV Expert Summer Passion Fluid | | | | | |
|---|---|---|---|---|---|
| Phase | Ingredients | INCI | Weight % | | Function |
| | | | a | b | |
| C | Water, demin. | Aqua | 10.00 | 10.00 | |
| | Eusolex 232 | Phenylbenzimidazole Sulfonic Acid | 3.50 | 3.50 | UV-B filter |
| | Tris Amino Ultra Pur | Tromethamine | qs | qs | |
| D | Perfume | Parfum | qs | qs | Fragrance |
| | Preservative | | qs | qs | Preservative |

| Performance (BASF sunscreen simulator) | a | b |
|---|---|---|
| SPF in silico | 60.8 | 56.7 |
| UVA-PF in silico | 21.9 | 32.2 |

| Manufacturing Process |
|---|
| Heat up phase B without Rheocare XG.<br>Heat up phase A to 75°C under stirring.<br>At 75°C add Rheocare XG to phase B under homogenizer.<br>Add phase A into B under stirring, homogenize.<br>Cool down to room temperature under continuous stirring, then add phase C previously mixed. Adjust pH value to at least 7. |

| Example B16: Sun Care Face Cream. Tinosorb A2B line | | | | | |
|---|---|---|---|---|---|
| Phase | Ingredients | INCI | Weight % | | Function |
| | | | a | b | |
| A | Eumulgin® Prisma | Disodium Cetearyl Sulfosuccinate | 1.50 | 1.50 | Emulsifier (O/W) |
| | Cetiol® B | Dibutyl Adipate | 15.00 | 15.00 | Emollient |
| | Cetiol® AB | C12-15 Alkyl Benzoate | 12.00 | 12.00 | Emollient |
| | Lanette® O | Cetearyl Alcohol | 2.50 | 2.50 | Consistency agent |
| | Cutina® HVG | Hydrogenated Vegetable Glycerides | 2.00 | 2.00 | Emollient |
| | Sensiva SC 50 | Ethylhexylglycerin | 0.50 | 0.50 | Humectant |
| | Parsol 1789 | Butyl Methoxydibenzoylmethane | 3.00 | 3.00 | UV-A filter |
| | UV-TRIOX-01 | | 3.00 | | |
| | UV-TRIOX-02 | | | 3.00 | |
| | Uvinul® T 150 | Ethylhexyl Triazone | 2.50 | 2.50 | UV-B filter |
| B | Water, demin. | Aqua | qs. 100 | qs. 100 | |
| | Glycerin | Glycerin | 3.00 | 3.00 | Humectant |
| | Cosmedia® SP | Sodium Polyacrylate | 1.00 | 1.00 | Rheology modifier |
| | Edeta® BD | Disodium EDTA | 0.20 | 0.20 | Complexing agent |
| | Keltrol CG-RD | Xanthan Gum | 0.15 | 0.15 | Rheology modifier |
| C | Eusolex 232 | Phenylbenzimidazole Sulfonic Acid | 1.50 | 1.50 | UV-B filter |
| | Water, demin. | Aqua | 5.00 | 5.00 | |
| | Tris Amino Ultra Pur | Tromethamine | qs | qs | |
| D | Preservative | | qs | qs | Preservative |
| | Orgasol Caresse | Polyamide-5 | 0.50 | 0.50 | |
| | Orgasol2002 D NAT COS | Nylon-12 | 0.50 | 0.50 | Skin feel modifier |
| E | Tinosorb® M | Methylene Bis-Benzotriazolyl Tetramethylbutylphenol (nano), Aqua, Decyl Glucoside, Propylene Glycol, Xanthan Gum | 8.00 | 8.00 | Broad spectrum UV filter |
| | Tinosorb® A2B | Tris-Biphenyl Triazine (nano), Aqua, Decyl Glucoside, Butylene Glycol, Disodium Phosphate, Xanthan Gum | 8.00 | 8.00 | UV-B/A II filter |

| Performance (BASF sunscreen simulator) | a | b |
|---|---|---|
| SPF in silico | 39.7 | 37.8 |
| UVA-PF in silico | 16.7 | 18.4 |

| Manufacturing Process |
|---|
| Heat up phase A to 80°C.<br>Heat up phase B to 80°C.<br>Incorporate phase A into phase B under stirring, homogenize. Add phase C previously mixed.<br>Cool down under stirring to room temperature.<br>Finally add the ingredients of D and E under stirring. Continue stirring for a while. |

| Example B17: High Efficiency Index Lotion | | | | | |
|---|---|---|---|---|---|
| Phase | Ingredients | INCI | Weight % | | Function |
| | | | a | b | |
| A | Cremophor® GS 32 | Polyglyceryl-3 Distearate | 4.00 | 4.00 | Emulsifier (O/W) |
| | Cutina® PES | Pentaerythrityl Distearate | 0.70 | 0.70 | Consistency agent |
| | Lanette® O | Cetearyl Alcohol | 0.50 | 0.50 | Consistency agent |
| | Cetiol® AB | C12-15 Alkyl Benzoate | 8.00 | 8.00 | Emollient |
| | Cetiol® B | Dibutyl Adipate | 8.00 | 8.00 | Emolient |
| | Cetiol® C 5 | Coco-Caprylate | 6.00 | 6.00 | Emollient |
| | Myritol® 312 | Caprylic/Capric Triglyceride | 2.00 | 2.00 | Emollient |
| | UV-TRIOX-01 | | 3.00 | | |
| | UV-TRIOX-02 | | | 3.00 | |
| | Uvinul® T 150 B | Ethylhexyl Triazone | 2.00 | 2.00 | UV-B filter |
| B | Water, demin. | Aqua | qs. 100 | qs. 100 | |
| | Glycerin | Glycerin | 3.00 | 3.00 | Humectant |
| | Lanette® E | Sodium Cetearyl Sulfate | 1.50 | 1.50 | Emulsifier (O/W) |
| | Keltrol CG-RD | Xanthan Gum | 0.30 | 0.30 | tabilizer |
| | Edeta® BD | Disodium EDTA | 0.20 | 0.20 | Complexing agent |

(continued)

| Example B17: High Efficiency Index Lotion | | | | | |
|---|---|---|---|---|---|
| Phase | Ingredients | INCI | Weight % | | Function |
| | | | a | b | |
| C | Tinosorb® M | Methylene Bis-Benzotriazolyl Tetramethylbutylphenol (nano), Aqua, Decyl Glucoside, Propylene Glycol, Xanthan Gum | 12.00 | 12.00 | Broad spectrum UV filter |
| | Tinosorb® A2B | Tris-Biphenyl Triazine (nano), Aqua, Decyl Glucoside, Butylene Glycol, Disodium Phosphate, Xanthan Gum | 6.00 | 6.00 | UV-B / A II filter |
| D | Orgasol Caresse | Polyamide-5 | 2.00 | 2.00 | |
| | Preservative | | qs | qs | |

| Performance (BASF sunscreen simulator) | a | b |
|---|---|---|
| SPF in silico | 45.9 | 44.4 |
| UVA-PF in silico | 15.3 | 17.6 |

| Manufacturing Process |
|---|
| Heat up part A to 80°C.<br>Heat up part B without Lanette E and Xanthan Gum to 80°C.<br>Add Xanthan Gum and Lanette E in this order under Ultra-Turrax to Part B.<br>Add Part A into B under Ultra-Turrax. Cool down under stirring to RT. Finally add the ingredients of C and D under stirring, homogenize. |

| Example B18: Common Stabilized UVA with Tinosorb A2B | | | | | |
|---|---|---|---|---|---|
| Phase | Ingredients | INCI | Weight % | | Function |
| | | | a | b | |
| A | Emulgade® PL 68/50 | Cetearyl Glucoside, Cetearyl Alcohol | 4.00 | 4.00 | Emulsifier (O/W) |
| | Eumulgin® BA 25 | Beheneth-25 | 1.50 | 1.50 | Emulsifier (O/W) |
| | Lanette® O | Cetearyl Alcohol | 1.00 | 1.00 | Consistency agent |
| | Cutina® PES | Pentaerythrityl Distearate | 1.00 | 1.00 | Consistency agent |
| | Cetiol® B | Dibutyl Adipate | 8.00 | 8.00 | Emollient |
| | Cetiol® CC | Dicaprylyl Carbonate | 3.00 | 3.00 | Emollient |
| | Uvinul® N 539T | Octocrylene | 10.00 | 10.00 | UV-B filter |
| | Parsol 1789 | Butyl Methoxydibenzoylmethane | 5.00 | 5.00 | UV-A filter |
| | Neo Heliopan OS | Ethylhexyl Salicylate | 5.00 | 5.00 | UV-B filter |
| | UV-TRIOX-01 | | 1.50 | | |
| | UV-TRIOX-02 | | | 1.50 | |
| B | Water, demin. | Aqua | qs. 100 | qs. 100 | |
| | Glycerin | Glycerin | 3.00 | 3.00 | Humectant |
| | Mais PO4 PH "B" | Distarch Phosphate | 1.00 | 1.00 | Skin feel modifier |
| | Keltrol CG-RD | Xanthan Gum | 0.30 | 0.30 | Rheology modifier |
| C | Tinosorb® A2B | Tris-Biphenyl Triazine (nano), Aqua, Decyl Glucoside, Butylene Glycol, Disodium Phosphate, Xanthan Gum | 6.00 | 6.00 | UV-B / A II filter |
| | Tinosorb® M | Methylene Bis-Benzotriazolyl Tetramethylbutylphenol (nano), Aqua, Decyl Glucoside, Propylene Glycol, Xanthan Gum | 2.00 | 2.00 | Broad spectrum UV filter |
| D | Xiameter PMX-0245 Cyclopentasiloxane | Cyclopentasiloxane | 2.00 | 2.00 | Emollient |
| | Orgasol 2002 EXD NAT COS | Nylon-12 | 2.00 | 2.00 | Skin feel modifier |
| | Perfume | Parfum | qs | qs | Fragrance |
| | Preservative | | qs | qs | Preservative |

| Performance (BASF sunscreen simulator) | a | b |
|---|---|---|
| SPF in silico | 50.3 | 49.8 |
| UVA-PF in silico | 22.2 | 23.5 |

| Manufacturing Process |
|---|
| Heat phase A and part B to 80°C under stirring. Add phase A into B under stirring, homogenize. Cool down to room temperature under stirring. Add phase C and D under stirring. Continue stirring. |

Example B19: Light Feeling Sun Care Cream Tinosorb A2B

| Phase | Ingredients | INCI | Weight % | | | | Function |
|---|---|---|---|---|---|---|---|
| | | | a | b | c | d | |
| A | Eumulgin® B 1 | Ceteareth-12 | 1.50 | 1.50 | 1.50 | 1.50 | Emulsifier (O/W) |
| | Cetiol® B | Dibutyl Adipate | 5.00 | 5.00 | 5.00 | 5.00 | Emollient |
| | Cetiol® CC | Dicaprylyl Carbonate | 3.00 | 3.00 | 3.00 | 3.00 | Emollient |
| | Uvinul® MC 80 | Ethylhexyl Methoxycinnamate | 7.50 | 7.50 | 7.50 | 7.50 | UV-B filter |
| | Uvinul® A Plus | Diethylamino Hydroxybenzoyl Hexyl Benzoate | 5.00 | 5.00 | 5.00 | 5.00 | UV-A filter |
| | Tinosorb® S | Bis-Ethylhexyloxy-phenol Methoxyphenyl Triazine | | 1.00 | | 1.00 | |
| | UV-TRIOX-01 | | 5.00 | 5.00 | | | |
| | UV-TRIOX-02 | | | | 5.00 | 5.00 | |
| B | Water, demin. | Aqua | qs. 100 | qs. 100 | qs. 100 | qs. 100 | |
| | Glycerin | Glycerin | 3.00 | 3.00 | 3.00 | 3.00 | Humectant |
| | Edeta® BD | Disodium EDTA | 0.20 | 0.20 | 0.20 | 0.20 | Complexing agent |
| C | Tinovis® ADE | Sodium Acrylates Copolymer, Hydrogenated Polydecene, PPG-1 Trideceth-6 | 1.70 | 1.70 | 1.70 | 1.70 | Rheology modifier |
| D | Tinosorb® A2B | Tris-Biphenyl Triazine (nano), Aqua, Decyl Glucoside, Butylene Glycol, Disodium Phosphate, Xanthan Gum | 5.00 | 5.00 | 5.00 | 5.00 | UV-B / A II filter |
| E | Xiameter PMX-0246 Cyclohexasiloxane | Cyclohexasiloxane, Cyclopentasiloxane | 2.00 | 2.00 | 2.00 | 2.00 | |
| | Dow Corning 9701 Cosmetic Powder | Dimethicone/Vinyl Dimethicone Crosspolymer, Silica | 1.00 | 1.00 | 1.00 | 1.00 | |
| F | Orgasol 2002 EXD NAT COS Typ S | Nylon-12 | 1.00 | 1.00 | 1.00 | 1.00 | |
| | Techpolymer MBP 8 | Polymethyl Methacrylate | 1.00 | 1.00 | 1.00 | 1.00 | |
| | Protectol® PE | Phenoxyethanol | qs | qs | qs | qs | Preservative |
| | Perfume | Parfum | qs | qs | qs | qs | Fragrance |

| Performance (BASF sunscreen simulator) | a | b | c | d |
|---|---|---|---|---|
| SPF in silico | 37.9 | 41.3 | 38.1 | 41.5 |
| UVA-PF in silico | 13.0 | 14.3 | 16.8 | 18.2 |

| Specifications | |
|---|---|
| Adjust ph value to (25°C) | 5.0 - 5.5 |
| Viscosity (Brookfield; DV-III Ultra; spindle RV06; 10 rpm; 20°C) | 15000 - 25000 mPas |

| Manufacturing Process |
|---|
| Heat phase A and phase B to 75°C<br>At 75°C add phase A into phase B under homogenizer.<br>Add phase C under homogenizer, to thick up the formulation.<br><br>Cool down to room temperature under stirring.<br>At least add ingridients of phase D, phase E and phase F. |

| Example B20: la Boite de Pandore Sun Care Cream | | | | | |
|---|---|---|---|---|---|
| Phase | Ingredients | INCI | Weight % | | Function |
| | | | a | b | |
| A | Eumulgin® SG | Sodium Stearoyl Glutamate | 2.00 | 2.00 | Emulsifier (O/W) |
| | Lanette® 18 | Stearyl Alcohol | 1.00 | 1.00 | Consistency agent |
| | Tegosoft XC | Phenoxyethyl Caprylate | 8.00 | 8.00 | Emollient |
| | Cetiol® B | Dibutyl Adipate | 5.00 | 5.00 | Emollient |
| | Finsolv EB | Ethylhexyl Benzoate | 5.00 | 5.00 | |
| | Crodamol LL | Lauryl Lactate | 3.00 | 3.00 | Emollient |
| | Uvinul® A Plus | Diethylamino Hydroxybenzoyl Hexyl Benzoate | 10.00 | 10.00 | UV-A filter |
| | Uvinul® T 150 | Ethylhexyl Triazone | 3.00 | 3.00 | UV-B filter |
| | UV-TRIOX-01 | | 5.00 | | |
| | UV-TRIOX-02 | | | 5.00 | |
| B | Water, demin. | Aqua | qs.100 | qs.100 | |
| | Glycerin | Glycerin | 3.00 | 3.00 | Humectant |
| | Cosmedia® SP | Sodium Polyacrylate | 0.60 | 0.60 | Rheology modifier |
| | Keltrol CG-RD | Xanthan Gum | 0.50 | 0.50 | Stabilizer |
| | Edeta® BD | Disodium EDTA | 0.20 | 0.20 | Complexing agent |

(continued)

| Example B20: la Boite de Pandore Sun Care Cream | | | | | |
|---|---|---|---|---|---|
| Phase | Ingredients | INCI | Weight % | | Function |
| | | | a | b | |
| C | Irgasol Caresse | Polyamide-5 | 1.50 | 1.50 | |
| | Preservative | | qs | qs | Preservative |

| Performance (BASF sunscreen simulator) | a | b |
|---|---|---|
| SPF in silico | 30.3 | 28.4 |
| UVA-PF in silico | 25.2 | 30.6 |

| Manufacturing Process |
|---|
| Heat phase A and B to 80°C.<br>Add phase A into B, homogenize.<br>Cool to room temperature then add phase C under stirring. Continue stirring for a while. |

| Example B21: la Boite de Pandore Sun Care Cream 2 | | | | | | | |
|---|---|---|---|---|---|---|---|
| Phas e | Ingredients | INCI | Weight % % | | | | Function |
| | | | a | b | c | d | |
| A | Eumulgin® SG | Sodium Stearoyl Glutamate | 2.00 | 2.00 | 2.00 | 2.00 | |
| | Lanette® 18 | Stearyl Alcohol | 1.00 | 1.00 | 1.00 | 1.00 | |
| | Tegosoft XC | Phenoxyethyl Caprylate | 8.00 | 8.00 | 8.00 | 8.00 | |
| | Cetiol® B | Dibutyl Adipate | 5.00 | 5.00 | 5.00 | 5.00 | |
| | Finsolv EB | Ethylhexyl Benzoate | 5.00 | 5.00 | 5.00 | 5.00 | |
| | Exceparl LM-LC | Lauryl Lactate | 5.00 | 5.00 | 5.00 | 5.00 | |
| | Uvinul® A Plus | Diethylamino Hydroxybenzoyl Hexyl Benzoate | 10.00 | 10.00 | | | |
| | Uvinul® T 150 | Ethylhexyl Triazone | 3.50 | 3.50 | | | |
| | UV-TRIOX-01 | | 3.00 | | | | |
| | UV-TRIOX-02 | | | 3.00 | | | |
| | Tinosorb® S | Bis-Ethylhexyloxy-phenol Methoxyphenyl Triazine | | | | | |

(continued)

| Example B21: la Boite de Pandore Sun Care Cream 2 | | | | | | | |
|---|---|---|---|---|---|---|---|
| Phas e | Ingredients | INCI | Weight % % | | | | Function |
| | | | a | b | c | d | |
| B | Water, demin. | Aqua | qs.100 | qs.100 | qs. 100 | qs. 100 | |
| | Glycerin | Glycerin | 3.00 | 3.00 | 3.00 | 3.00 | Humectant |
| | Keltrol CG-RD | Xanthan Gum | 0.50 | 0.50 | 0.50 | 0.50 | Stabilizer |
| | Edeta® BD | Disodium EDTA | 0.20 | 0.20 | 0.20 | 0.20 | Complexing agent |
| C | Eusolex 232 | Phenylbenzimidazole Sulfonic Acid | 3.00 | 3.00 | 3.00 | 3.00 | UV-B filter |
| | Water, demin. | Aqua | 5.00 | 5.00 | 5.00 | 5.00 | |
| | Tris Amino Ultra Pur | Tromethamine | qs | qs | qs | qs | |
| D | Orgasol Caresse | Polyamide-5 | 1.50 | 1.50 | 1.50 | 1.50 | |
| | Preservative | | qs | qs | qs | qs | Preservative |

| Performance (BASF sunscreen simulator) | a | b | c | d |
|---|---|---|---|---|
| SPF in silico | 48.0 | 48.0 | 51.4 | 51.4 |
| UVA-PF in silico | 25.0 | 28.6 | 26.4 | 30.1 |

| Manufacturing Process |
|---|
| Heat phase A and B to 80°C. Add phase A into B, homogenize. Add phase C under ultra-turrax device. Cool to room temperature then add phase D under stirring. Continue stirring for a while. |

| Example B22: Dry Touch Stabilized UVA Cream Gel | | | | | |
|---|---|---|---|---|---|
| Phase | Ingredients | INCI | Weight % | | Function |
| | | | a | b | |
| A | Eumulgin® B 2 | Ceteareth-20 | 2.00 | 2.00 | Emulsifier (O/W) |
| | Emulgade® Sucro | Sucrose Polystearate, Hydrogenated Polyisobutene | 2.50 | 2.50 | Emulsifier (O/W) |
| | Cetiol® AB | C12-15 Alkyl Benzoate | 9.00 | 9.00 | Emollient |
| | Cetiol® B | Dibutyl Adipate | 5.00 | 5.00 | Emollient |
| | Uvinul® N 539T | Octocrylene | 10.00 | 10.00 | UV-B filter |
| | Neo Heliopan OS | Ethylhexyl Salicylate | 5.00 | 5.00 | UV-B filter |
| | Parsol 1789 | Butyl Methoxydibenzoylmethane | 5.00 | 5.00 | UV-A filter |
| | Uvasorb HEB | Diethylhexyl Butamido Triazone | 4.00 | 4.00 | UV-B filter |
| | UV-TRIOX-01 | | 3.50 | | |
| | UV-TRIOX-02 | | | 3.50 | |
| | Uvinul® MC 80 | Ethylhexyl Methoxycinnamate | 0.60 | 0.60 | UV-B filter |
| B | Water, demin. | Aqua | qs. 100 | qs. 100 | |
| | Keltrol CG-RD | Xanthan Gum | 0.30 | 0.30 | Stabilizer |
| | Edeta® BD | Disodium EDTA | 0.20 | 0.20 | Complexing agent |
| C | Xiameter PMX-0246 Cyclohexasiloxane | Cyclohexasiloxane, Cyclopentasiloxane | 2.00 | 2.00 | Emollient |
| D | Preservative | | qs | qs | Preservative |

| Performance (BASF sunscreen simulator) | a | b |
|---|---|---|
| SPF in silico | 44.4 | 44.0 |
| UVA-PF in silico | 12.9 | 14.3 |

| Manufacturing Process |
|---|
| Heat part A to 80°C. Heat part B to 80°C. Add part A into part B under stirring, homogenize.<br>Cool down to room temperature under stirring. Then add part C, D and E continue stirring for a while. |

Example B23: Velour Delight

Optimal suncare protection for daily use

Single UV absorber - dedicated to all skin types, including sensitive and children skins

| Phase | Ingredients | INCI | Weight % | | | | | | Function |
|---|---|---|---|---|---|---|---|---|---|
| | | | a | b | c | d | e | f | |
| A | Cetiol® CC | Carbonate | 5.00 | 5.00 | 5.00 | 5.00 | 5.00 | 5.00 | Emollient |
| | Cetiol® B | Dibutyl Adipate | 4.00 | 4.00 | 4.00 | 4.00 | 4.00 | 4.00 | Emollient |
| | Cetiol® C 5 | Coco-Caprylate | 3.00 | 3.00 | 3.00 | 3.00 | 3.00 | 3.00 | Emollient |
| | Dow Corning 9041 Silicone | Dimethicone, Dimethicone | 1.50 | 1.50 | 1.50 | 1.50 | 1.50 | 1.50 | Skin feel modifier |
| | Elastomer Blend | Cross-polymere | | | | | | | |
| | Sensiva SC 50 | Ethylhexylglycerin | 0.50 | 0.50 | 0.50 | 0.50 | 0.50 | 0.50 | Auxiliary |
| | UV-TRIOX-01 | | 10.00 | 20.00 | 30.00 | | | | Broad spectrum UV filter |
| | UV-TRIOX-02 | | | | | 10.00 | 20.00 | 30.00 | |
| B | Water, demin. | Aqua | qs. 100 | qs. 100 | qs. 100 | qs. 100 | qs. 100 | qs. 100 | |
| | Glycerin | Glycerin | 3.00 | 3.00 | 3.00 | 3.00 | 3.00 | 3.00 | Humecta nt |
| | Protectol® PE | Phenoxyethanol | 1.00 | 1.00 | 1.00 | 1.00 | 1.00 | 1.00 | Preservative |
| | Eumulgin® SG | Sodium Stearoyl Glutamate | 0.80 | 0.80 | 0.80 | 0.80 | 0.80 | 0.80 | Emulsifier (O/W) |
| | Rheocare® XG (Europe only) | Xanthan Gum | 0.20 | 0.20 | 0.20 | 0.20 | 0.20 | 0.20 | Stabilizer |
| | Edeta® BD | Disodium EDTA | 0.20 | 0.20 | 0.20 | 0.20 | 0.20 | 0.20 | Complexing agent |
| C | Tinovis® ADE | Sodium Acrylates Copolymer, Hydrogenated Polydecene, PPG-1 Trideceth-6 | 1.00 | 1.00 | 1.00 | 1.00 | 1.00 | 1.00 | Rheology modifier |
| D | Orgasol Caresse | Polyamide-5 | 3.00 | 3.00 | 3.00 | 3.00 | 3.00 | 3.00 | |
| E | Perfume | Parfum | qs | qs | qs | qs | qs | qs | Fragranc e |

(continued)

| Example B23: Velour Delight<br>Optimal suncare protection for daily use<br>Single UV absorber - dedicated to all skin types, including sensitive and children skins | | | | | | | | | |
|---|---|---|---|---|---|---|---|---|---|
| Phase | Ingredients | INCI | Weight % | | | | | | Function |
| | | | a | b | c | d | e | f | |
| F | Neutrol® TE | Tetrahydroxy-propyl Ethylenediamine | qs | qs | qs | qs | qs | qs | Neutralizing agent |

| Performance (BASF sunscreen simulator) | a | b | c | d | e | f |
|---|---|---|---|---|---|---|
| SPF in silico | 16.0 | 33.0 | 51.7 | 15.8 | 33.1 | 51.2 |
| UVA-PF in silico | 0.52 | 0.41 | 0.36 | 1.20 | 1.17 | 1.12 |

| Extinction diagrams (BASF sunscreen simulator)F | |
|---|---|
| Ex. B23c with 30% UV-TRIOX-01<br>Label SPF 50 | 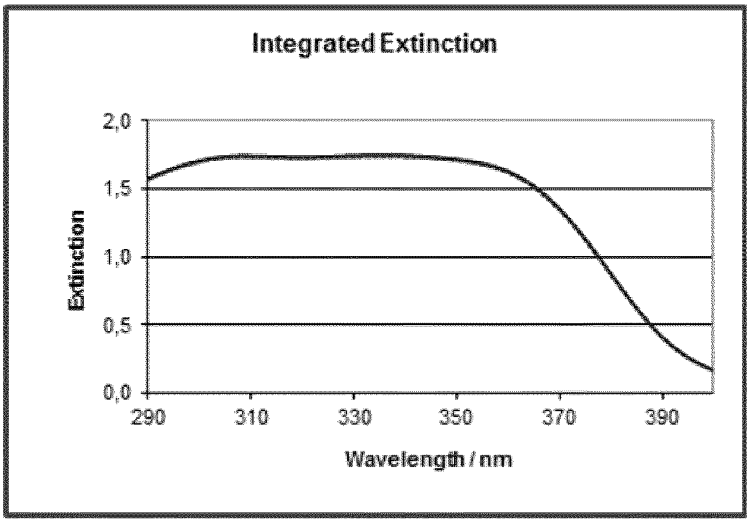 |
| Ex. B23f with 30% UV-TRIOX-02<br>Label SPF 50 | 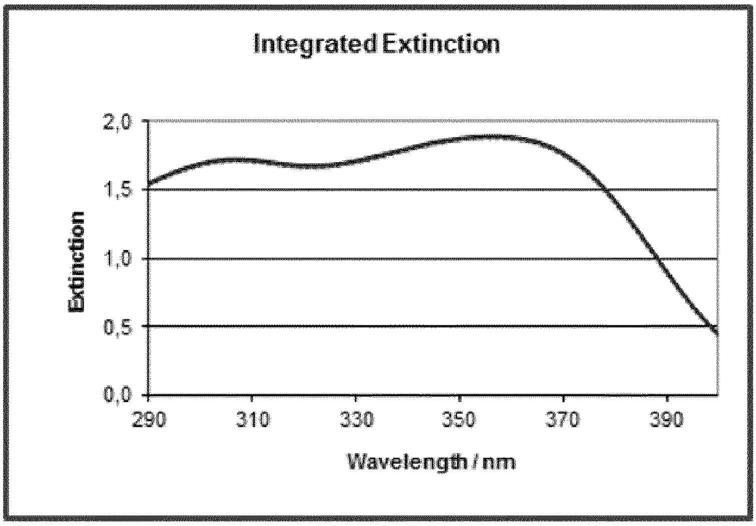 |

(continued)

| Extinction diagrams (BASF sunscreen simulator)F | |
|---|---|
| Ex. B23a with 10% UV-TRIOX-01<br>Label SPF 15 | 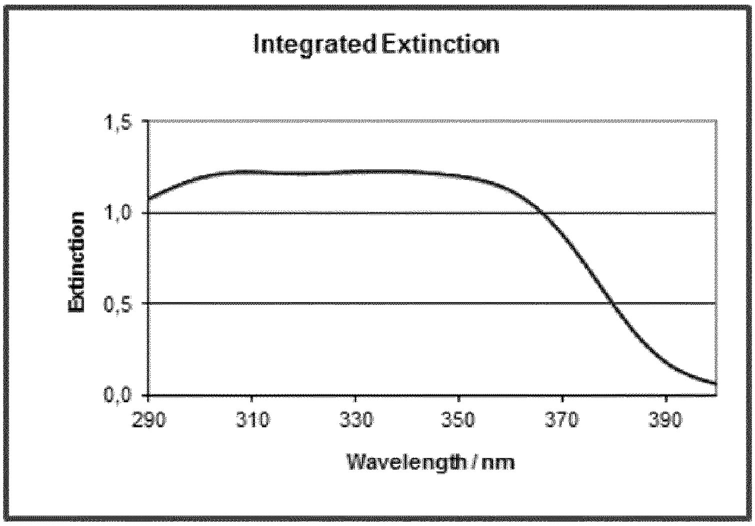 |
| Ex. B23d with 10% UV-TRIOX-02<br>Label SPF 15 | 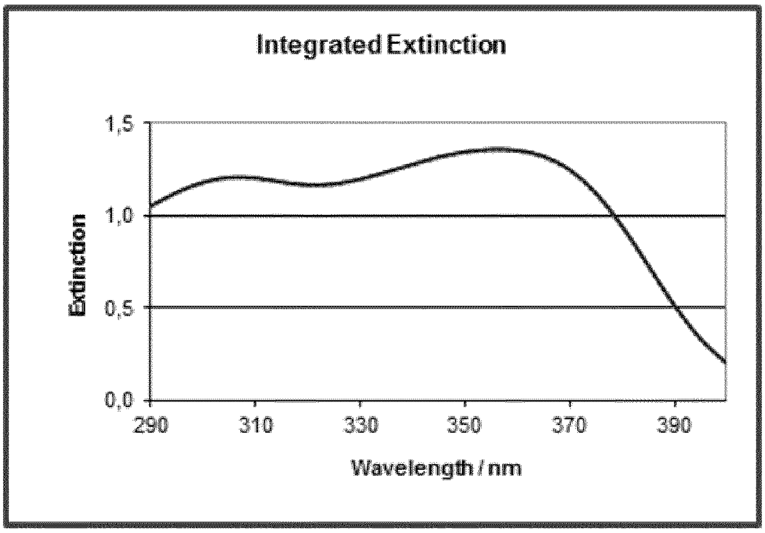 |

| Example B24: Daily Care - Spectral Homeostasis | | | | | | | | |
|---|---|---|---|---|---|---|---|---|
| Phase | Ingredients | INCI | Weight % | | | | | |
| | | | a | b | c | d | e | f |
| A | Emulgade PL 68/50 | Ceateryl Glucoside (and) Cetearyl Alcohol | 4.00 | 4.00 | 4.00 | 4.00 | 4.00 | 4.00 |
| | Eumulgin prisma | Disodium Cetearyl Sulfosuccinate | 1.50 | 1.50 | 1.50 | 1.50 | 1.50 | 1.50 |
| | Cetiol AB | C12-15 Alkyl Benzoate | 8.00 | 8.00 | 8.00 | 8.00 | 8.00 | 8.00 |
| | Cetiol B | Dibutyl Adipate | 8.00 | 8.00 | 8.00 | 8.00 | 8.00 | 8.00 |
| | DUB DIS | Diisopropyl Sebacate | 5.00 | 5.00 | 5.00 | 5.00 | 5.00 | 5.00 |
| | Cetiol® C 5 | Coco-Caprylate | 4.00 | 4.00 | 4.00 | 4.00 | 4.00 | 4.00 |
| | Cutina® PES | Pentaerythrityl Distearate | 1.00 | 1.00 | 1.00 | 1.00 | 1.00 | 1.00 |
| | Uvinul® A Plus | Diethylamino Hydroxybenzoyl Hexyl Benzoate | 8.00 | 8.00 | 8.00 | 8.00 | 8.00 | 8.00 |
| | Uvinul® T 150 | Ethylhexyl Triazone | 2.00 | 2.00 | 2.00 | 2.00 | 2.00 | 2.00 |
| | UV-TRIOX-01 | | 10.00 | 20.00 | 30.00 | | | |
| | UV-TRIOX-02 | | | | | 10.00 | 20.00 | 30.00 |
| B | Water, demin. | Aqua | qs. 100 | qs. 100 | qs. 100 | qs. 100 | qs. 100 | qs. 100 |
| | Glycerin 85% | Glycerin | 3.00 | 3.00 | 3.00 | 3.00 | 3.00 | 3.00 |
| | Edeta® BD | Disodium EDTA | 0.20 | 0.20 | 0.20 | 0.20 | 0.20 | 0.20 |
| | Rheocare® XG | Xanthan Gum | 0.40 | 0.40 | 0.40 | 0.40 | 0.40 | 0.40 |
| | Protectol® PE | Phenoxyethanol | 1.00 | 1.00 | 1.00 | 1.00 | 1.00 | 1.00 |
| | Rheocare® C Plus | Carbomer | 0.15 | 0.15 | 0.15 | 0.15 | 0.15 | 0.15 |
| C | Tinosorb® S Aqua | Aqua, Bis-Ethyl-hexyloxyphenol Methoxyphenyl Triazine, Polymethyl Methacrylate, Sodium Laureth Sulfate, Aminomethyl Propanol | 7.50 | 7.50 | 7.50 | 7.50 | 7.50 | 7.50 |
| | C1332 | | 20.00 | 20.00 | 20.00 | 20.00 | 20.00 | 20.0 0 |
| | Spheron 1500 | Silica | 1.00 | 1.00 | 1.00 | 1.00 | 1.00 | 1.00 |
| | Orgasol Caresse | Polyamide-5 | 2.00 | 2.00 | 2.00 | 2.00 | 2.00 | 2.00 |
| | 50% solution Neutrol® TE | Tetrahydroxypro-pyl Ethylenediamine | 0.80 | 0.80 | 0.80 | 0.80 | 0.80 | 0.80 |

| Performance (BASF sunscreen simulator) | a | b | c | d | e | f |
|---|---|---|---|---|---|---|
| SPF in silico | 52.2 | 72.6 | 92.7 | 51.4 | 71.2 | 90.6 |
| UVA-PF in silico | 61.8 | 62.3 | 63.1 | 81.9 | 99.4 | 114.6 |

Example B24:

| Phase | Ingredients | INCI | Weight % | | | | | | | |
|---|---|---|---|---|---|---|---|---|---|---|
| | | | a | b | | | | | | |
| A | Emulgade® 165 | Glyceryl Stearate, PEG-100 Stearate | 2.00 | 2.00 | | | | | | |
| | Cetiol® B | Dibutyl Adipate | 3.00 | 3.00 | | | | | | |
| | Finsolv TPP | C12-15 Alkyl Benzoate,) Dipropylene Glycol Dibenzoate, PPG-15 Stearyl Ether Benzoate | 3.00 | 3.00 | 3.00 | 3.00 | 3.00 | 3.00 | 3.00 | 3.00 |
| | Lanette® 18 | Stearyl Alcohol | 1.00 | 1.00 | 1.00 | 1.00 | 1.00 | 1.00 | 1.00 | 1.00 |
| | Lanette® O | Cetearyl Alcohol | 1.00 | 1.00 | 1.00 | 1.00 | 1.00 | 1.00 | 1.00 | 1.00 |
| | Uvinul® N 539T | Octocrylene | 10.00 | 10.00 | 10.00 | 10.00 | 10.00 | 10.00 | 10.00 | 10.00 |
| | Neo Heliopan OS | Ethylhexyl Salicylate | 5.00 | 5.00 | 5.00 | 5.00 | 5.00 | 5.00 | 5.00 | 5.00 |
| | Eusolex 9020 | Butyl Methoxydibenzoylmethane | 4.00 | 4.00 | 4.00 | 4.00 | 4.00 | 4.00 | 4.00 | 4.00 |
| | UV-TRIOX-01 | | | | 5.00 | 12.00 | 1.00 | | | |
| | UV-TRIOX-02 | | | | | | | 5.00 | 12.00 | 1.00 |
| | Tinosorb® S | Bis-Ethylhexyloxyphenol Methoxyphenyl Triazine | 2.00 | 2.00 | 2.00 | 1.00 | 1.00 | 2.00 | 1.00 | 1.00 |
| B | MT-100 Z | Titanium Dioxide (nano), Aluminum Hydroxide, Stearic Acid | 2.70 | 2.70 | 2.70 | 2.70 | 2.70 | 2.70 | 2.70 | 2.70 |
| | Water, demin. | Aqua | 46.75 | 46.75 | 46.75 | 46.75 | 46.75 | 46.75 | 46.75 | 46.75 |
| | Amphisol K | Potassium Cetyl Phosphate | 2.80 | 2.80 | 2.80 | 2.80 | 2.80 | 2.80 | 2.80 | 2.80 |
| | Tinocare® GL | Sclerotium Gum | 1.00 | 1.00 | 1.00 | 1.00 | 1.00 | 1.00 | 1.00 | 1.00 |
| | Keltrol CG-RD | Xanthan Gum | 0.25 | 0.25 | 0.25 | 0.25 | 0.25 | 0.25 | 0.25 | 0.25 |
| C | Edeta® BD | Disodium EDTA | 0.20 | 0.20 | 0.20 | 0.20 | 0.20 | 0.20 | 0.20 | 0.20 |
| D | Xiameter PMX-0246 Cyclohexasiloxane | Cyclohexasiloxane, Cyclopentasiloxane | 2.00 | 2.00 | 2.00 | 2.00 | 2.00 | 2.00 | 2.00 | 2.00 |
| E | Tinovis® ADE | Sodium Acrylates Copolymer, Hydrogenated Polydecene, PPG-1 Trideceth-6 | 0.50 | 0.50 | 0.50 | 0.50 | 0.50 | 0.50 | 0.50 | 0.50 |
| F | Tinosorb® M | Methylene Bis-Benzotriazolyl Tetramethylbutylphenol (nano), Aqua, Decyl Glucoside, Propylene Glycol, Xanthan Gum | 0.00 | 10.00 | 6.00 | 0.00 | 0.00 | 6.00 | 0.00 | 0.00 |

(continued)

Example B24:

| Phase | Ingredients | INCI | Weight % | | | | | | | |
|---|---|---|---|---|---|---|---|---|---|---|
| | | | a | b | | | | | | |
| | Preservative | | qs | qs | qs | qs | qs | qs | qs | qs |
| G | Perfume | Parfum | qs | qs | qs | qs | qs | qs | qs | qs |
| | Sodium Hydroxide (10% solution) | Sodium Hydroxide | 0.80 | 0.80 | 0.80 | 0.80 | 0.80 | 0.80 | 0.80 | 0.80 |

| Performance (BASF sunscreen simulator) | a | b | c | d | e | f | g | h |
|---|---|---|---|---|---|---|---|---|
| SPF in silico | 32.9 | 51.7 | 53.9 | 50.9 | 31.5 | 51.1 | 50.4 | 31.6 |
| UVA-PF in silico | 16.2 | 30.1 | 24.3 | 21.1 | 15.2 | 32.1 | 31.0 | 16.0 |

| Manufacturing Process |
|---|
| Heat up phase A to 75 -80°C without Titanium Dioxide.<br>At 75°C, add Titanium Dioxide, homogenize until homogeneously dispersed.<br>Heat up phase B without Keltrol RD and Amphisol K.<br>At 75°C add Keltrol RD and Amphisol K to phase B and mix until homogeneous.<br>Incorporate phase A into phase B under homogenization (Ultra Turrax type device). Mix until homogeneous.<br>Cool down under stirring. At 60°C, add phase C, mix until homogeneous then add phase D and homogenize. Cool down to room temperature, then add phase E and mix until homogeneous. Add phase F, finally adjust pH with phase G. |

## Claims

1. UV absorber mixture comprising at least two compounds which refer to the reaction product of an oxirane mixture and a hydroxyphenyl-triazine compound.

2. UV absorber mixture according to claim 1, wherein the oxiranes correspond to formula

(1a) 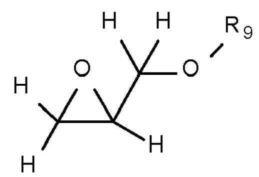

wherein

Rg is $C_1$-$C_{18}$alkyl; or $C_3$-$C_{12}$cycloalkyl.

3. UV absorber mixture according to claim 1 or 2 wherein the oxirane mixture comprises at least 2 different oxiranes.

4. UV absorber mixture according to any of the preceding claims, wherein the oxirane mixture comprises at least 3 different oxiranes.

5. UV absorber mixture according to any of the preceding claims, wherein the reaction product corresponds to formula

(1) 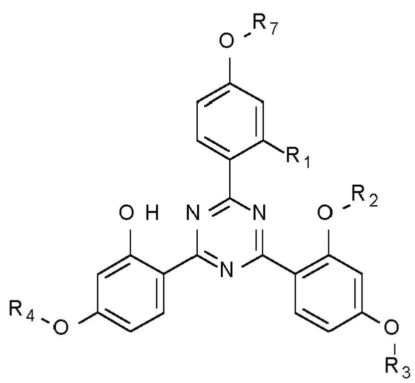 ,

wherein

$R_1$ is hydrogen; or -O-$R_6$;
$R_2$, $R_3$, $R_4$ and $R_6$, independently from each other are hydrogen; or a radical of formula

(1b) 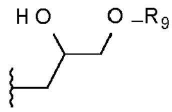 ,

wherein
Rg is $C_1$-$C_{18}$alkyl; or $C_3$-$C_{18}$cycloalkyl; and
$R_7$ is hydrogen; $C_1$-$C_{18}$alkyl; or $C_3$-$C_{12}$cycloalkyl;

wherein at least 2 of $R_2$, $R_3$, $R_4$ and $R_6$ are a radical of formula (1b); and wherein the UV absorber mixture comprises at least 2 different compounds of formula (1).

**6.** UV absorber mixture according to any of claims 1 to 5, wherein

$R_1$ is hydrogen.

**7.** UV absorber mixture according to any of claims 1 to 5, wherein

$R_1$ -O-$R_6$; and
$R_6$ is hydrogen; or a radical of formula

(1b) 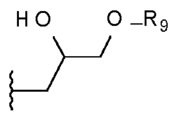 ,

wherein
Rg is $C_1$-$C_{18}$alkyl; or $C_3$-$C_{18}$cycloalkyl.

**8.** UV absorber mixture according to any of the preceding claims, wherein the reaction product corresponds to formula

(1) 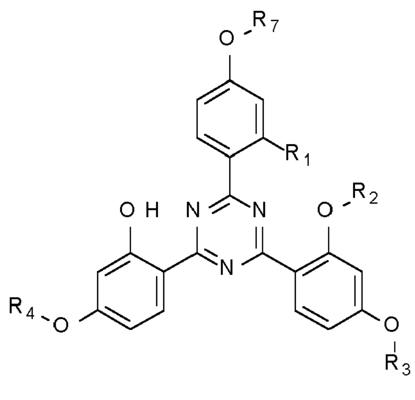 ,

wherein

$R_1$ is hydrogen; or hydroxy;
$R_2$ is hydrogen; or a radical of formula (1 b);
$R_3$ and $R_4$ are radical of formula (1b); and
$R_7$ is hydrogen; $C_1$-$C_{18}$alkyl; or $C_3$-$C_{12}$cycloalkyl.

**9.** UV absorber mixture according to any of the preceding claims, wherein said UV absorber mixture has an average molecular weight in the range of about 900 to about 5000

**10.** UV absorber mixture according to any of the preceding claims, wherein said UV absorber mixture has a polydispersity index of about 3 or less.

**11.** UV absorber mixture according to any of the preceding claims, wherein said UV absorber mixture has a solubility of >35% by weight in cosmetic solvents selected from Ethylhexyl Benzoate, C12-15 Alkyl Benzoate, Dibutyl Adipate, Dicaprylyl Carbonate, Dibutyl Sebacate, mixture Undecane and Tridecane (Cetiol Ultimate) and Cocoglycerides.

**12.** UV absorber mixture according to any of the preceding claims, wherein said UV absorber mixture has a solubility of >39% by weight in C12-15 Alkyl Benzoate, Dibutyl Adipate Dicaprylyl Carbonate.

**13.** UV absorber mixture according to any of the preceding claims, wherein said UV absorber mixture is amorphous at room temperature.

**14.** UV absorber mixture according to any of the preceding claims, wherein said UV absorber mixture is highly photostable (no degradation detectable after 50 MED irradiation of a $2mg/cm^2$ film containing 2% of the UV absorber according to claim 1).

**15.** Process for the preparation of the UV absorber mixture of formula (1) as defined in claim 1, which comprises the reaction of alkyl glycidyl ethers of formula (1c) with hydroxyphenyl triazines of formula (4) according to the following reaction scheme:

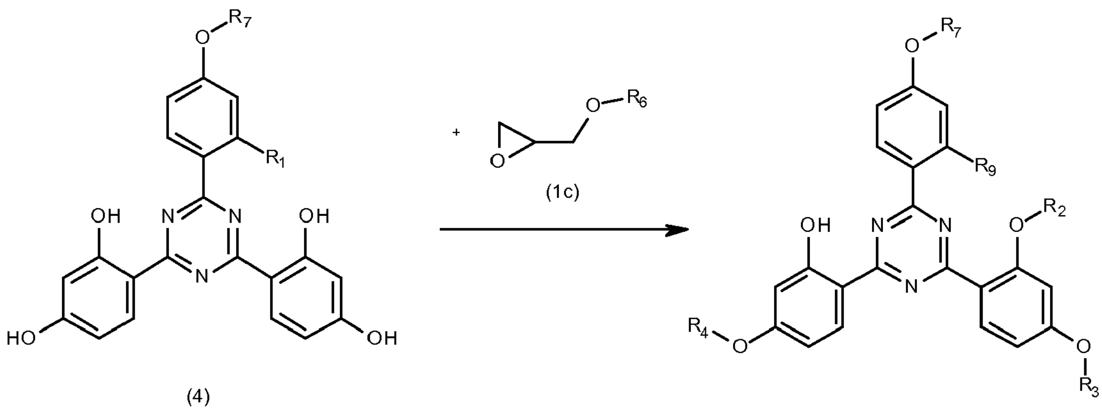

(1c)

(4)

(1)

wherein

$R_1$ is hydrogen; or hydroxy;
$R_2$, $R_3$ and $R_4$, independently from each other, are hydrogen; or a radical of formula

(1b) 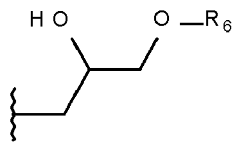

$R_6$ is $C_1$-$C_{18}$alkyl;
$R_7$ is hydrogen; or $C_1$-$C_{18}$alkyl;
Rg is hydrogen; or -O-$R_{10}$;
$R_{10}$ is hydrogen; or a radical of formula (1b);

wherein at least 2 of the radicals $R_2$, $R_3$, $R_4$ and $R_{10}$ are a radical of formula (1b); and wherein the UV absorber mixture comprises at least 2 different compounds of formula (1).

**16.** Cosmetic composition, comprising

(a) from 30 to 90 % b.w. of a continuous water phase,

(b) from 5 to 50 % of a discontinuous oil phase homogeneously distributed in said water phase and
(c) from 1 to 20 % b.w. of at least one emulsifier selected from

($c_1$) oil-in-water emulsifier;
($c_2$) w/o emulsifier and
($c_3$) w/Si emulsifier

(d) said oil phase comprising 1-70% by weight of the UV absorber mixture as defined claim (1).

**17.** Cosmetic composition according to claim 16, wherein said composition is substantially free of a UV absorber compound other than the UV absorber mixture as defined in claim (1).

**18.** Cosmetic composition according to claim 16 or 17, wherein the UV absorber mixture as defined in claim (1) is present in an amount effective to provide said composition with an SPF of about 10 or greater.

**19.** Cosmetic composition according to any of claims 16 to 18, wherein the emulsifier ($c_1$), ($c_2$) or ($c_3$) is an anionic emulsifier.

**20.** Cosmetic composition according to any of claims 16 to 18, wherein the wherein the emulsifier ($c_1$), ($c_2$) or ($c_3$) is a nonionic emulsifier.

**21.** The composition according to any of claims 16 to 20 comprising additional cosmetic UV absorbers.

**22.** Use of the cosmetic composition according to any of claims 16 to 21 for minimizing the appearance of dysesthetic feeling of the skin.

**23.** Use of the UV absorber mixture as defined in any of claims 1 to 14 for the enhancement of the solubility of other UV absorbers present in the cosmetic composition as defined in any of claims 16 to 21.

Europäisches Patentamt
European Patent Office
Office européen des brevets

# EUROPEAN SEARCH REPORT

Application Number
EP 18 16 8380

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 98/03489 A1 (CIBA GEIGY AG [CH]; TOAN VIEN VAN [CH]; VALET ANDREAS [DE]; HAYOZ PASC) 29 January 1998 (1998-01-29) * pages 1, 60, 82 and 83; table 3; compounds 18-20, 27-29, 32, 33 * ----- | 1-4 | INV. C07D251/24 A61K31/53 |
| X | DE 196 17 770 A1 (AGFA GEVAERT AG [DE]) 14 November 1996 (1996-11-14) * page 9; compound IV5 * ----- | 1-3 | |
| X | WO 2006/131466 A1 (CIBA SC HOLDING AG [CH]; VOGEL THOMAS [DE]; BRAIG ADALBERT [DE]; SCHAE) 14 December 2006 (2006-12-14) * claims 4, 21 * ----- | 1-4 | |
| X,D | EP 0 775 698 A1 (CIBA GEIGY [CH]) 28 May 1997 (1997-05-28) * claims 1, 12, 13; examples 4, 11; compound 103 * ----- | 1-23 | |
| X | US 2008/156227 A1 (SU CHING-YIE [TW] ET AL) 3 July 2008 (2008-07-03) * claim 8 * ----- | 1-3 | TECHNICAL FIELDS SEARCHED (IPC) |
| X | EP 0 704 437 A2 (CIBA GEIGY AG [CH]) 3 April 1996 (1996-04-03) * examples 3, 7 * ----- | 1-4 | C07D A61K |
| X | WO 2015/168389 A1 (CYTEC IND INC [US]; GUPTA RAM B [US]; ENG JERRY MON HEI [US]; KHAWAM F) 5 November 2015 (2015-11-05) * page 1, line 5 - line 6; claim 1; table 1; compounds B-15, B-22 * ----- | 1-4,6,7, 9-14, 16-23 | |

The present search report has been drawn up for all claims

2

| Place of search | Date of completion of the search | Examiner |
|---|---|---|
| Munich | 26 September 2018 | Moriggi, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number

EP 18 16 8380

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 18 16 8380

The Search Division considers that the present European patent application does not comply with the requirements of unity of invention and relates to several inventions or groups of inventions, namely:

1. claims: 5, 8, 15(completely); 1-4, 6, 7, 9-14, 16-23(partially)

   a UV absorber mixture comprising at least two compounds of formula (1) according to current claim 5, the process for making it and its use in a cosmetic composition

---

2. claims: 1-4, 6, 7, 9-14, 16-23(all partially)

   a UV absorber mixture comprising at least two compounds which refer to the reaction product of an oxirane mixture and a hydroxyphenyl-triazine compound, other than the mixture of formula (1), and its use in a cosmetic composition

---

## ANNEX TO THE EUROPEAN SEARCH REPORT ON EUROPEAN PATENT APPLICATION NO. EP 18 16 8380

This annex lists the patent family members relating to the patent documents cited in the above-mentioned European search report. The members are as contained in the European Patent Office EDP file on
The European Patent Office is in no way liable for these particulars which are merely given for the purpose of information.

26-09-2018

| Patent document cited in search report | | Publication date | Patent family member(s) | | | Publication date |
|---|---|---|---|---|---|---|
| WO 9803489 | A1 | 29-01-1998 | AU | 3620497 | A | 10-02-1998 |
| | | | BR | 9710730 | A | 17-08-1999 |
| | | | CA | 2258523 | A1 | 29-01-1998 |
| | | | CZ | 9900142 | A3 | 14-04-1999 |
| | | | DE | 69719990 | D1 | 24-04-2003 |
| | | | DE | 69719990 | T2 | 11-09-2003 |
| | | | EP | 0912531 | A1 | 06-05-1999 |
| | | | EP | 1266889 | A2 | 18-12-2002 |
| | | | ES | 2192688 | T3 | 16-10-2003 |
| | | | JP | 2000515141 | A | 14-11-2000 |
| | | | KR | 20000067910 | A | 25-11-2000 |
| | | | TW | 440564 | B | 16-06-2001 |
| | | | US | 6369267 | B1 | 09-04-2002 |
| | | | US | 2002094320 | A1 | 18-07-2002 |
| | | | WO | 9803489 | A1 | 29-01-1998 |
| DE 19617770 | A1 | 14-11-1996 | DE | 19617770 | A1 | 14-11-1996 |
| | | | US | 5935773 | A | 10-08-1999 |
| WO 2006131466 | A1 | 14-12-2006 | AR | 054200 | A1 | 06-06-2007 |
| | | | BR | PI0613486 | A2 | 11-01-2011 |
| | | | CN | 101193870 | A | 04-06-2008 |
| | | | EP | 1888539 | A1 | 20-02-2008 |
| | | | ES | 2439917 | T3 | 27-01-2014 |
| | | | JP | 5220592 | B2 | 26-06-2013 |
| | | | JP | 2008545771 | A | 18-12-2008 |
| | | | KR | 20080020677 | A | 05-03-2008 |
| | | | TW | 200706537 | A | 16-02-2007 |
| | | | US | 2009136768 | A1 | 28-05-2009 |
| | | | US | 2013143051 | A1 | 06-06-2013 |
| | | | WO | 2006131466 | A1 | 14-12-2006 |
| EP 0775698 | A1 | 28-05-1997 | AT | 250589 | T | 15-10-2003 |
| | | | AU | 717998 | B2 | 06-04-2000 |
| | | | BR | 9605663 | A | 18-08-1998 |
| | | | DE | 19543730 | A1 | 28-05-1997 |
| | | | DE | 59610730 | D1 | 30-10-2003 |
| | | | EP | 0775698 | A1 | 28-05-1997 |
| | | | EP | 1380583 | A2 | 14-01-2004 |
| | | | ES | 2206552 | T3 | 16-05-2004 |
| | | | IL | 119666 | A | 26-07-2000 |
| | | | JP | 4104185 | B2 | 18-06-2008 |
| | | | JP | H09188666 | A | 22-07-1997 |
| | | | KR | 970027060 | A | 24-06-1997 |
| | | | MX | 205723 | B | 18-12-2001 |
| | | | NZ | 299786 | A | 24-09-1998 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO. EP 18 16 8380

This annex lists the patent family members relating to the patent documents cited in the above-mentioned European search report. The members are as contained in the European Patent Office EDP file on
The European Patent Office is in no way liable for these particulars which are merely given for the purpose of information.

26-09-2018

| Patent document cited in search report | Publication date | Patent family member(s) | Publication date |
|---|---|---|---|
| | | SG 45516 A1 | 16-01-1998 |
| | | US 5955060 A | 21-09-1999 |
| | | ZA 9609807 B | 23-05-1997 |
| US 2008156227 A1 | 03-07-2008 | TW 200829637 A | 16-07-2008 |
| | | US 2008156227 A1 | 03-07-2008 |
| EP 0704437 A2 | 03-04-1996 | AT 224880 T | 15-10-2002 |
| | | BR 9503460 A | 27-02-1996 |
| | | CA 2154626 A1 | 28-01-1996 |
| | | CZ 9501925 A3 | 14-02-1996 |
| | | DE 69528331 D1 | 31-10-2002 |
| | | DE 69528331 T2 | 15-05-2003 |
| | | EP 0704437 A2 | 03-04-1996 |
| | | ES 2181760 T3 | 01-03-2003 |
| | | JP 3965603 B2 | 29-08-2007 |
| | | JP H0853427 A | 27-02-1996 |
| | | SK 93595 A3 | 07-02-1996 |
| | | TW 308602 B | 21-06-1997 |
| | | US 5543518 A | 06-08-1996 |
| | | US 5556973 A | 17-09-1996 |
| | | US 5637706 A | 10-06-1997 |
| | | US 5648488 A | 15-07-1997 |
| | | US 5675004 A | 07-10-1997 |
| | | US 5681955 A | 28-10-1997 |
| | | US 5684070 A | 04-11-1997 |
| WO 2015168389 A1 | 05-11-2015 | BR 112016025445 A2 | 15-08-2017 |
| | | CA 2947699 A1 | 05-11-2015 |
| | | CN 106471049 A | 01-03-2017 |
| | | EP 3137540 A1 | 08-03-2017 |
| | | JP 2017518430 A | 06-07-2017 |
| | | KR 20160147279 A | 22-12-2016 |
| | | TW 201546242 A | 16-12-2015 |
| | | US 2015315465 A1 | 05-11-2015 |
| | | US 2017349730 A1 | 07-12-2017 |
| | | WO 2015168389 A1 | 05-11-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 775698 A
- WO 2010136360 A
- WO 2011003774 A
- WO 0341675 A
- WO 2002039974 A
- DE 10229995
- EP 1371358 A
- EP 1371357 A
- EP 1371356 A
- DE 10138496
- US 20040247536 A
- DE 102007035567
- WO 2009012871 A
- DE 1165574 A
- DE 2024051 A
- FR 2252840 A


### Non-patent literature cited in the description


- *IP.com Journal,* 2009, vol. 9 (1B), 17
- **TODD et al.** of suitable volatile silicones may in addition be found. *Cosm. Toil.,* 1976, vol. 91, 27
- *J. Soc. Cosm. Chem.,* 1973, vol. 24, 281
- *J. Pharm. Pharmacol.,* 1975, vol. 26, 531
- **R. LOCHHEAD.** *Cosm. Toil.,* 1993, vol. 108, 95
- Kosmetische Farbemittel. Farbstoffkommission der Deutschen Forschungsgemeinschaft. Verlag Chemie, 1984, 81-106
- *SOFW-Journal,* vol. 139 (7-2013), 7-14
- **R. M. SAYRE ; P. P. AGIN ; G. J. LEVEE ; E. MARLOWE.** A comparison of in vivo and in vitro testing of sunscreening formulas. *Photochem. Photobiol.,* 1979, vol. 29, 559-566
- **COLIPA.** International Sun Protection Factor (SPF) Test Method. European Cosmetic Toiletries and Perfumery Association - COLIPA, 2003
- **B. L. DIFFEY ; J. ROBSON.** A new substrate to measure sunscreen protection factors throughout the ultraviolet spectrum. *J. Soc. Cosmet. Chem.,* 1989, vol. 40, 127-133
- **A. F. MCKINLAY ; B. L. DIFFEY.** A reference action spectrum for ultraviolet-induced erythema inhuman skin. *CIE Journal,* 1987, vol. 6, 17-22
- **J. J. O'NEILL.** Effect of film irregularities on sunscreen efficacy. *J.Pharm. Sci.,* 1984, vol. 73, 888-891
- Prediction of Sun Protection Factors and UV-A Parameters by Calculation of UV Transmissions Through Sunscreen Films of Inhomogenous Surface Structure, Chapter 44 in: Sunscreens: Regulations and Commercial Development. **B. HERZOG.** Cosmetic Science and Technology Series. Taylor & Francis Group, 2005, vol. 28, 881-900
- **L. FERRERO ; M. PISSAVINI ; S. MARGUERIE ; L. ZASTROW.** Efficiency of a continuous height distribution model of sunscreen film geometry to predict a realistic sun protection factor. *J. Cosmet. Sci.,* 2003, vol. 54, 463-481
- **M. WLOKA ; R. F. M. LANGE ; H. FLÖSSER-MÜLLER.** An in vitro SPF Screening Approach Considering the Photostability of the UV Filters. *Proc. Int. Sun Protection Conference,* 2005
- **B. HERZOG ; S. MONGIAT ; K. QUASS ; C. DESHAYES.** Prediction of Sun Protection Factors and UVA Parameters by Using a Calibrated Step Film Model. *J. Pharm. Sc.,* 2004, vol. 93, 1780-1795
- SPF Testing in Europe, The International SPF Test Method, Chapter 39 in: Sunscreens: Regulations and Commercial Development. **M. BROWN.** Cosmetic Science and Technology Series. Taylor & Francis Group, 2005, vol. 28, 779-806
- **COLIPA.** method for the in vitro determination of UVA protection provided by sunscreen products. The European Cosmetics Toiletry and Perfumery Association - COLIPA, 2007
- Models for the Calculation of Sun Protection Factors and Parameters Characterizing the UVA Protection Ability of Cosmetic Sunscreens. **BERND HERZOG.** Colloids in Cosmetics and Personal Care. Wiley-VCH, 2008, vol. 4